# EUROPEAN PATENT SPECIFICATION

(11) **EP 4 308 114 B1**
(45) Date of publication and mention of the grant of the patent: **11.02.2026**
(21) Application number: 22716203.9
(22) Date of filing: 17.03.2022
(51) Int. Cl.: A61K 31/405, A61K 45/06, A61K 31/00, A61K 31/451, A61P 37/08, C07K 16/28, A61K 39/00

(54) **C5AR1 INHIBITORS FOR TREATING HYPERSENSITIVITY REACTIONS TO TAXANES**
C5AR1 INHIBITOREN ZUR BEHANDLUNG VON HYPERSENSITIVITÄTSREAKTIONEN AUF TAXANE
C5AR1 INHIBITEURS POUR LE TRAITEMENT DES RÉACTIONS D'HYPERSENSIBILITÉ AUX TAXANES

(30) Priority: 17.03.2021 EP 21163292; 23.12.2021 EP 21217382
(43) Date of publication of application: 24.01.2024
(73) Proprietor: Dompé farmaceutici SpA, 20122 Milano (IT)
(72) Inventor: ALLEGRETTI, Marcello, 67100 L'Aquila (IT); ARAMINI, Andrea, 67100 L'Aquila (IT); AMENDOLA, Piergiorgio, 80131 Napoli (IT); BRANDOLINI, Laura, 67100 L'Aquila (IT); CESTA, Maria Candida, 67100 L'Aquila (IT); SIRICO, Anna, 80131 Napoli (IT)
(74) Representative: Dompé farmaceutici Spa
(86) International application number: PCT/EP2022/057007
(87) International publication number: WO 2022/195017

(56) References cited:
- WO-A1-01/30448
- WO-A1-98/53811
- WO-A2-2007/060215
- WO-A2-2019/115493
- NEENA I MARUPUDI, JAMES E HAN, KHAN W LI, VIOLETTE M RENARD, BETTY M TYLER& HENRY BREM: "Paclitaxel: a review of adverse toxicities and noveldelivery strategies", EXPERTOPINION ON DRUG SAFETY, 6:5, 609-621, DOI: 10.1517/14740338.6.5.609, 2007, pages 609 - 621
- OWEN A. HAWKSWORTH�, XARIA X. LI, LIAM G. COULTHARD�, ERNST J. WOLVETANGB, TRENT M. WOODRUFF': "New concepts on the therapeutic control of complement anaphylatoxin receptors", MOLECULAR IMMUNOLOGY, 2017
- MEDLER TERRY R ET AL: "Complement C5a Fosters Squamous Carcinogenesis and Limits T Cell Response to Chemotherapy", CANCER CELL, CELL PRESS, US, vol. 34, no. 4, 8 October 2018 (2018-10-08), pages 561, XP085501820, ISSN: 1535-6108, DOI: 10.1016/J.CCELL.2018.09.003

## Description

### FIELD OF THE INVENTION

The present invention relates to the prevention and treatment of hypersensitivity reactions to taxanes.

### STATE OF THE ART

Hypersensitivity reactions (HSRs) are unpredictable reactions characterized by clinical manifestations resembling an allergic reaction even in absence of evidence of an immunological mechanism. They can be life-threatening, may require or prolong hospitalization, and may necessitate changes in subsequent therapy. In general, a severe HSR is defined as any reaction severe enough to warrant discontinuation of the drug.

HSRs can be allergic, involving an immune response mediated by immunoglobulins and/or T cells, or non allergic, without a specific immune response involved.

Clinically, HSRs are commonly classified as immediate or non-immediate/delayed depending on their onset with respect to the treatment that has caused them.

Immediate HSRs usually occur while the medication is being administered or within the first hour after drug exposure, are generally induced by an IgE-mediated mechanism and are usually non allergic. On the contrary, non-immediate HSRs occur from one hour after from the initial drug administration and are usuallly associated with a delayed T-cell-dependent type of allergic mechanism.

However, there are limitations to the mechanistic classification above because other factors, such as route of administration, role of drug metabolites, and presence of cofactors or co-prescribed drugs, may accelerate or slow down the onset and/or the progression of a reaction (Demoly et al, Allergy 2014, 69: 420-437).

HSRs to chemotherapy and, in particular, to taxanes and platin agents, are very common and represent a serious threat to the treatment of oncologic patients, in some cases preventing the use of the first line, most effective drugs for their specific cancer.

The symptoms include cutaneous symptoms such as flushing, chest, back and abdominal pain as well as respiratory symtoms. More severe reactions are characterized also by oxygen desaturation and/or hypotension.

Although it has now been established that HSRs to platins are IgE-mediated, mechanisms of HSRs to taxanes remain to be defined.

The taxanes used in clinical practice are paclitaxel, docetaxel and cabazitaxel.

They exert their antineoplastic activity by interfering with the dynamics of microtubules (components of the cell cytoskeleton), thereby inhibiting mitosis and inducing apoptosis in cells undergoing the division process.

Paclitaxel (Taxol^{®}), also available for clinical use as albumin-bound paclitaxel (N*ab*^{™}-paclitaxel; Abraxane^{®}), is a natural compound originally isolated in the 1960s from the bark of the Pacific yew tree (Taxus brevifolia). It is used for the treatment of ovarian, breast, non-small cell lung cancers, and AIDS-related Kaposi's sarcoma.

Docetaxel (Taxotere^{®}) is a semisynthetic taxane that was developed as a more soluble and easier to produce alternative to paclitaxel. It is used in the treatment of breast, ovarian, prostate, head and neck, nonsmallcell lung carcinoma and gastric adenocarcinoma.

Cabazitaxel (Jevtana^{®}) is a semisynthetic taxane that was developed to overcome tumor drug resistance to paclitaxel and docetaxel.

These molecules are all poorly soluble in water and thus, in order to allow their intravenous administration, require the addition to their formulation of emulsifying agents, in particular of Cremophor^{®} EL (mixture of polyoxyethylated triglycerides ) for paclitaxel formulations and polysorbate 80 (Tween 80) for the more soluble docetaxel and carbazitaxel.

The majority of HSRs to taxanes occur during the first or second infusion of the drug and usually are immediate HSRs, developing within minutes of starting the infusion, suggesting that these are not dependent on prior sensitization (Picard et al, Clinic Rev Allerg Immunol 2015, 49:177-191).

It has been hypothesized the emulsifying agents used to solubilize paclitaxel and the other taxanes are responsible for HSRs through complement activation and anaphylotoxins production with subsequent mast cells and basophils secretory response. However, also a solvent free paclitaxel formulation, N*ab*^{™}paclitaxel, can still induce HSRs in a significant percentage of treated patients, thus suggesting a direct sensitization mechanism.

Furthemore, recent findings have also raised the possibility that taxanes-induced HSRs may be IgE-mediated, supporting the use of skin prick tests to identify patients at risk (Prieto et al, J Investig Allergol Clin Immunol 2010, 20: 170-171; Picard et al, J Allergy Clin Immunol 2016, 137:1154-64), and suggesting that taxanes may induce basophil activation through a non-IgE-mediated mechanism (Picard et al, J Clinic Rev Allerg Immunol 2015, 49:177-191).

This lack of a clear understanding of the main causes of HSRs due to taxanes has led in practice to a wide diversity of management strategies. Premedication with corticosteroids and antihistamine compounds is used as preventive treatment in order to reduce the incidence of reactions. However, notwithstanding the premedication, a high percentage of patients experiences HSRs, with a variable incidence depending on the used chemotherapeutic drug (10% with paclitaxel, 5% with docetaxel, 4% with N*ab*^{™}-paclitaxel and around 1% with cabazitaxel), including severe, life threatening HSRs (Picard et al, Immunol Allergy Clin An Am 2017, 37: 679-693).

During the last decade, the development of drug desensitization and graded challenging protocols has allowed to re-introduce taxanes in some of patients experiencing HSRs.

However, there is still a high need to better understand the mechanism of HSRs to taxanes, and to develop a sound clinical approach to prevent HSRs in patients treated with these drugs.

The anaphylatoxins (AT) C3a, C5a and C5a-desArg are pro-inflammatory polypeptides generated after proteolytic cleavage of C3 and C5 in response to complement activation. C5a acts primarily through C5a receptor 1 (C5aR1), exerting pro-inflammatory and immunomodulatory functions. Dysregulation of the C5a-C5aR1 axis has been implicated in various immune disorders.

C5aR1 is expressed on a broad range of cell types, including all cells of myeloid origin (neutrophils, eosinophils, monocytes, macrophages, dendritic cells, mast cells), lymphocytes, and non-myeloid cells, such as lung, liver, kidney, skin, and central nervous system (CNS) cells. Document WO98/53811 provides a method of preventing or reducing hypersensitivity and allergic reactions in human patients receiving taxane therapy by administering the taxane orally instead of intravenously.

Document WO01/30448 confirms that evidence exists that paclitaxel itself provokes acute hypersensitivity reactions. To reduce such allergy, prodrugs of paclitaxel have been produced, such as the methylpyridinium mesylate salt.

### SUMMARY OF THE INVENTION

The present inventors have now surprisingly found that taxanes are able to bind the C5a receptor C5aR1 and activate the same signaling pathways triggered by C5a binding to this receptor, thereby causing anaphylactic reactions that account for HSRs related to administration of these drugs.

Accordingly, a first aspect of the invention is a C5aR1 inhibitor for use in the prevention or treatment of hypersensitivity reactions (HSRs) to taxanes in an individual.

A second aspect of the invention is a pharmaceutical composition comprising a C5aR1 inhibitor and at least one inert pharmaceutically acceptable excipient, for use in the prevention or treatment of hypersensitivity reactions to taxanes in an individual.

### BRIEF DESCRIPTION OF THE FIGURES

Figure 1 shows the concentration over time, expressed in resonance units (RU), of C5aR1 bound to the sensorchip, obtained by C5aR1 capture via c-Myc-tag, as described in Example 1a). The difference between the baseline RU before (starting baseline) and after (final baseline) injection of C5AR1-cMyc onto the sensorchip corresponds to a stable attachment of 77 RU of the receptor to the surface.
Figure 2 shows the concentration over time, expressed in resonance units (RU), of C5a bound to C5aR1 immobilised on the sensorchip, as described in Example 1b).
Figure 3 shows the concentration, expressed in resonance units (RU), of Paclitaxel bound to C5aR1 immobilised on the sensorchip, as described in Example 1c).
Figure 4 shows the concentration over time, expressed in resonance units (RU), of Paclitaxel bound to C5aR1 immobilised on the sensorchip, measured at different Paclitaxel concentrations (from 4.06 µM to 37.5 µM), as described in Example 1c).
Figure 5 shows the steady-state concentration, expressed in resonance units (RU), of Paclitaxel bound to C5aR1 immobilised on the sensorchip, measured as a function Paclitaxel concentration, as described in Example 1c).
Figure 6 shows the concentration over time, expressed in resonance units (RU), of C5aR2 bound to the microchip, obtained by C5aR2 capture via His-tag, as described in Example 2a). The difference between the baseline RU before (starting baseline) and after (final baseline) injection of C5aR2-His onto the sensorchip corresponds to a stable attachment of 569 RU of the receptor to the surface.
Figure 7 shows the concentration over time, expressed in resonance units (RU), of C5a bound to C5aR2 immobilised on the sensorchip, as described in Example 2b).
Figure 8 shows the concentration over time, expressed in resonance units (RU), of Paclitaxel bound to C5aR2 immobilised on the sensorchip, measured at different Paclitaxel concentrations (from 4.06 µM to 37.5 µM), as described in Example 2c). Paclitaxel is not able to bind to receptor at any of the concentrations tested.
Figure 9 shows the concentration over time, expressed in resonance units (RU), of Paclitaxel bound to C5a on the sensorchip, as described in Example 3b). Paclitaxel is not able to bind to C5a at the concentration tested.
Figure 10 shows profiling cytokines in RAW264.7 supernatants treated with Paclitaxel for 24h, as described in Example 4. The numbers on the membranes indicate each cytokine revealed in the assay and reported in the histograms (1: MCP-1/CCL2; 2: C5a; 3: TNF alpha; 4: MIP-1 alpha/CCL3; 5: MI-1 beta/CCL4; 6: MIP2/CXCL2; 7: IL-1ra; 8: Rantes/CCL5).
Figure 11 shows the cytokine levels expressed as pixel density, in RAW264.7 supernatants treated with C5a for Paclitaxel for 24h, and measured as described in Example 4.
Figure 12 shows the results of Real Time PCR analysis for IL-8 relative mRNA expression in F11 cells treated for 20h with 10 nM Paclitaxel (PAC) or 50nM C5a (50nMC5a).
Figure 13 shows the results of the mean pixel density for each cytokine released by murine RAW264 macrophages (Panels A and B), and human macrophages (Panel C and D), measured as described in Example 6, untreated (CTR) or treated with C5a alone (C5a) or C5A in combination with a C5AR1 antibody (anti C5AR+C5A), with Avacopan (AV+C5A) or with DF3966A (DF+C5A), paclitaxel alone (PAC) or paclitaxel in combination with a C5AR1 antibody (anti C5AR+PAC), with Avacopan (AV+PAC) or with DF3966A (DF+PAC).

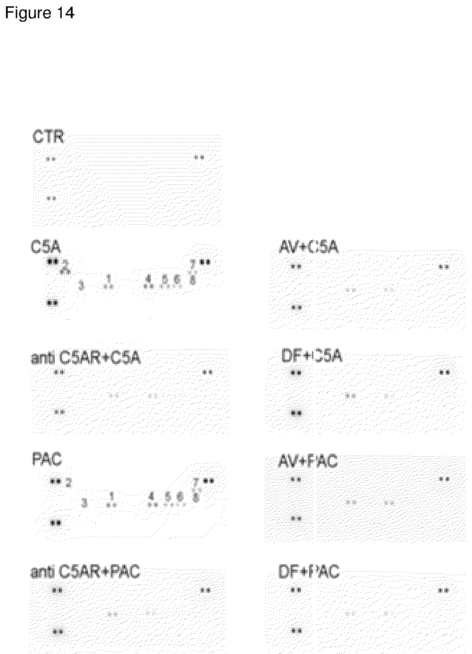

Figure 14 shows the membranes with each cytokine released by murine RAW264 macrophages revealed in the assay as described in Example 6 and reported in the histograms of Figure 13.
Figure 15 shows the results of the mean pixel density for each cytokine released in human macrophages (Panel A and B), measured as described in Example 6, untreated (CTR) or treated with C5a alone (C5a) or C5A in combination with a C5AR1 antibody (anti C5AR+C5A), with Avacopan (AV+C5A) or with DF3966A (DF+C5A), paclitaxel alone (PAC) or paclitaxel in combination with a C5AR1 antibody (anti C5AR+PAC), with Avacopan (AV+PAC) or with DF3966A (DF+PAC).

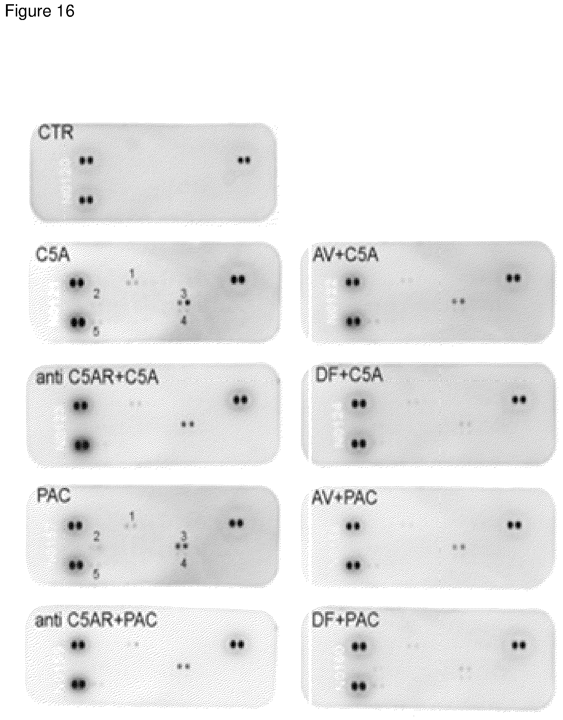

Figure 16 shows the membranes with each cytokine released by human macrophages revealed in the assay as described in Example 6 and reported in the histograms of Figure 15.
Figure 17 shows the results of the permeability degree score (Panel A) and the vascular leakage (Panel B) calculated through the spectrophotometer (O.D.), in mice treated with PEG/TWEEN alone (PEG/TWEEN) paclitaxel in DMSO/PEG/TWEEN (PAC in PEG/TWEEN), Cremophor EL alone (Cremophor) or paclitaxel in Cremophor EL (PAC in Cremophor).
Figure 18 shows the results of the in vivo experiment in mice treated with Cremophor alone (Cremophor) or paclitaxel dissolved in Cremophor in combination with the vehicle used for DF3966A (PAC in Cremophor +Veh DF), with DF3966A (PAC in Crem +DF), with the vehicle used for Avacopan (PAC in Cremophor +Veh AVA) or with Avacopan (PAC in Crem +AVA). Efficacy results are shown based on 4 different parameters: permeability degree (Panel A), vascular leakage (Panel B) histamine and SC5B-9 (Panel C and D), measured as described in Example 8.

### DEFINITIONS

According to the present invention, the term "prevention" refers to administration to an individual to obtain partial or complete prevention of a disorder or pathological event, before this is established or occurs.

According to one embodiment of the invention, prevention is a complete prevention, wherein the disorder or pathological event is completely blocked.

According to an alternative embodiment of the invention, prevention is a partial prevention, wherein the development of the disorder or pathological event is delayed and/or reduced in severity.

According to the present invention, the term "treatment" refers to complete reversal or reduction of severity or progression of a disorder or pathological event, after this is established or occurs.

According to the present invention, the term "individual" refers to a human or an animal being, preferably to a human being.

According to the present invention, the term "hypersensitivity reaction" or "HSR" is used to refer to an unpredictable adverse reaction to a drug, characterized by a clinical manifestation resembling an allergic reaction, even in absence of evidence of an immunological mechanism.

### DETAILED DESCRIPTION OF THE INVENTION

A first aspect of the present invention is a C5aR1 inhibitor for use in the prevention or treatment of a hypersensitivity reaction (HSR) to a taxane in an individual.

Preferably, said taxane is selected from paclitaxel, N*ab*^{™}-paclitaxel, docetaxel and cabazitaxel.

Said individual is preferably a subject diagnosed with a cancer for which the treatment of election is a taxane. Preferably, said cancer is selected from ovarian, breast, non-small cell lung, prostate, head and neck carcinoma, gastric adenocarcinoma, and AIDS-related Kaposi's sarcoma.

According to a first embodiment, said C5aR1 inhibitor is for use in the prevention of a hypersensitivity reaction to a taxane in said individual and the inhibitor is administered one or more times prior to each administration of said taxane.

Preferably, said C5aR1 inhibitor is administered one or more times in the frametime between 30 minutes and 24 hours before administering the taxane to the individual. More preferably, it is administered between 30 minutes and 2 hours before administering the taxane to the individual.

According to this embodiment, the above individual is preferably a subject at risk of developing a hypersensitivity reaction. Preferably, said individual is a cancer patient that has had a previous hypersensitivity reaction episode to a taxane, has resulted positive in a skin prick test for sensitivity to said taxane, and/or has a history of atopy.

According to this embodiment, the C5aR1 inhibitor may also be administered in combination with one or more corticosteroid and/or antihistamine drugs. In some cases, the C5aR1 inhibitor and the corticosteroids and/or antihistamines are administered substantially simultaneously. Alternatively, the C5aR1 inhibitor and the corticosteroid and/or antihistamine drugs are administered sequentially, all in the frametime between 30 minutes and 24 hours before administering the taxane to the individual.

Preferably, said corticosteroid and/or antihistamine drugs are those routinely used for premedication treatment for taxanes. Preferably, the C5aR1 inhibitor is administered in combination with dexamethasone, diphenhydramine and ranitidine. More preferably, it is administered in combination with oral administration of dexamethasone (8 mg) between 12 and 6 h before infusion of the taxane and intravenous administration of diphenhydramine (2 mg) and ranitidine (50 mg) 30 min before infusion of the taxane. According to an alternative embodiment, said C5aR1 inhibitor is for use in the acute treatment of a hypersensitivity reaction to a taxane in said individual and the inhibitor is administered at the onset of the symptoms of the hypersensitivity reaction. In this instance, the treatment of said individual with the taxane is also immediately stopped. According to this embodiment, the C5aR1 inhibitor may be administered in combination with other suitable treatments, preferably corticosteroids, dopamine and/or antihistamines. In most severe cases, also epinephrine and/or oxygen supplementation may be administered.

The term "C5aR1 inhibitor" in accordance with the present invention means any compound that interacts with C5aR1 and prevents its binding to C5a or that blocks the signalling of C5aR1 upon binding of C5a. Preferably, said inhibitor of C5aR1 is selected from C5aR1 competitive antagonists, anti-C5aR1 antibodies able to block the C5a binding sites on the receptor and C5aR1 non-competitive allosteric inhibitors.

Many different C5aR1 inhibitors according to the above definition have been developed and are well known to the skilled man.

According to one preferred embodiment, said C5aR1 antagonists are selected from the group consisting of:
- (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide (Avacopan, Vynpenta^{®});
- N-Acetyl-L-phenylalanyl-L-ornithyl-L-prolyl-3-cyclohexyl-D-alanyl-L-tryptophyl-L-arginine-N-5.2-C-1.6-lactam (PMX-53);
- Acetylated phenylalanine-[ornithyl-proline-(D)cyclohexylalanine-tryptophyl-arginine];
- L-Alanyl-L-seryl-glycyl-L-alanyl-L-prolyl-L-alanyl-L-prolyl-glycyl-L-prolyl-L-alanyl-glycyl-L-prolyl-L-leucyl-L-arginyl-L-prolyl-L-methionyl-L-phenylalanine;
- N,N-Bis(1,3-benzodioxol-5-ylmethyl)-N-(1-butyl-2,4-diphenyl-1H-imidazol-5-ylmethyl)amine;
- N-[2-(4-Chlorophenyl)ethyl]-N-(1,4-dioxaspiro[4.5]dec-8-yl)-2-isobutylbenzamide;
- N-[2-(4-Chlorophenyl)ethyl]-N-(4-hydroxycyclohexyl)-1-benzothiophene-3-carboxamide;
- N-[2-(4-Chlorophenyl)ethyl]-N-(4-hydroxycyclohexyl)naphthalene-1-carboxamide;
- 2-(2-Ethyl-6-methylphenyl)-4-methoxy-N-(5-methoxy-2-methylphenyl)-5,6,7,8-tetrahydroquinolin-5-amine;
- 2-(2,6-Diethylphenyl)-N-ethyl-4-methoxy-N-(1-naphthyl)-5,6,7,8-tetrahydroquinolin-5-amine;
- N-[2,6-Dioxohexahydropyrimidin-4(S)-ylcarbonyl]-L-phenylalanyl-L-ornithyl-L-prolyl-5-methyl-L-norleucyl-4-fluoro-L-phenylalanyl-L-phenylalaninamide (JPE-1375; JSM-1375);
- N-(3-Phenylpropionyl)-L-ornithyl-L-prolyl-3-cyclohexyl-D-alanyl-L-tryptophyl-L-arginine N-5.1-C-1.5-lactam (PMX-205);
- N,N'-Bis(4-amino-2-methylquinolin-6-yl)urea (NSC12155);
- N-[4-(Dimethylamino)benzyl]-N-(4-isopropylphenyl)-7-methoxy-1,2,3,4-tetrahydronaphthalene-1-carboxamide hydrochloride (W54011);
- L-Phenylalanyl-L-ornithyl-L-prolyl-3-cyclohexyl-D-alanyl-L-tryptophyl-L-arginine N-5.2-C-1.6-cyclic peptide;
- (4aR,16aS)-6,18-Dihydroxy-23(S)-[2(S)-hydroxy-2-[2(R)-hydroxy-6(R)-methyl-5(R)-[2(S)-methylbutyl]tetrahydro-2H-pyran-2-yl]propionamido]-22(S)-isopropyl-7(S),19(R)-dimethyldocosahydro-13H,22H-dipyridazino[6,1-f:6',1'-o][1,4,7,10,13,16]oxapentaazacyclononadecine-5,7,11,17,20,24-hexanone (L-156602).

According to one preferred embodiment, said anti-C5aR1 antibodies are selected from the group consisting of:
- Avdoralimab (IPH-5401)
- MOR-044254;
- NOX-D20;
- Anti-C5aR1ab-C5-SiRNA;
- m20/70 mlgG2a.1;
- 3C5;
- 7F3.

According to one preferred embodiment, said non-competitive allosteric inhibitors are selected from:
(2*R*)-2-[3-(furan-2-carbonyl)phenyl]-*N*-[4-(trifluoromethyl)-1,3-thiazol-2-yl]propenamide (DF2427);
   and
compounds having general formula (I): 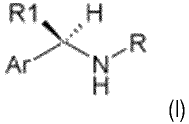
or a pharmaceutically acceptable salt thereof, wherein
   i) R is selected from:
      - 2 -thiazolyl or 2-oxazolyl, unsubstituted or substituted by a group selected from methyl, tert-butyl or trifluoromethyl group;
      - C(Ra)=N-W wherein W is linear or branched C₁-C₄ alkyl,
      - CORa, SORa, SO₂Ra, PORa, PO₂Ra,
      wherein
      **Ra** is selected from
      - C₁-C₅-alkyl, C₃-C₆-cycloalkyl, C₂-C₅-alkenyl, unsubstituted or substituted phenyl with a group selected from halogen, C₁-C₄-alkyl, C₁-C₄-alkoxy, halo-C₁-C₄-alkoxy, hydroxy, C₁-C₄-acyloxy, phenoxy, cyano, nitro, amino;
      - a heteroaryl group selected from pyridine, pyrimidine, pyrrole, thiophene, furane, indole, thiazole, oxazole, such heteroaryl being unsubstituted or substituted with a group selected from halogen, C₁-C₄-alkyl, C₁-C₄-alkoxy, halo-C₁-C₄-alkoxy, hydroxy, C₁-C₄- acyloxy, phenoxy, cyano, nitro, amino;
      - a α or β carboxyalkyl residue consisting of straight or branched C₁- C₆-alkyl, C₃-C₆-cycloalkyl, C₂-C₆-alkenyl, C₁-C₆-phenylalkyl, optionally substituted with a further carboxy (COOH) group;
      - an ω-aminoalkylamino group of formula (II): 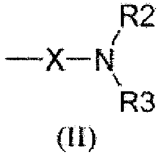 wherein in said formula (II)
         X is:
            - linear or branched C₁-C₆ alkylene, C₄-C₆ alkenylene, C₄-C₆ alkynylene, optionally substituted by: a) a CO₂R4 group, wherein R4 represents hydrogen or a linear or branched C₁-C₆ alkyl group or a linear or branched C₂-C₆ alkenyl group, or b) by a CONHR5 group wherein R5 represents hydrogen, linear or branched C₂- C₆ alkyl or an OR4 group, R4 being defined as above;
            - a (CH₂)ₘ-B-(CH₂)ₙ, group, optionally substituted by a CO₂R4 or CONHR5 group, as defined above, wherein a) B is an oxygen, or sulfur atom, or nitrogen atom optionally substituted by a C₁-C₄ alkyl group, m is zero or an integer from 2 to 3 and n is an integer from 2 to 3, or b) B is a CO, SO or CONH group, m is an integer from 1 to 3 and n is an integer from 2 to 3;
         **R2** and **R3** are independently hydrogen, linear or branched C₁-C₆ alkyl, optionally interrupted by an oxygen or sulfur atom, a C₃-C₇ cycloalkyl, C₃-C₆ alkenyl, C₃-C₆-alkynyl, aryl-C₁-C₃-alkyl, hydroxy-C₂-C₃-alkyl group; or
         **R2** and **R3** together with the N atom to which they are bound, form a 3-7 membered nitrogen heterocyclic ring of formula (III) 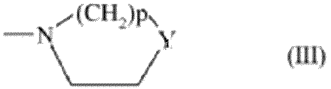 wherein
            Y represents:
               - a single bond, CH₂, O, S, or a N-R6 group, where R6 represents hydrogen, C₁-C₄ alkyl, C₁-C₄ acyl, unsubstituted or substituted phenyl with a group selected from halogen, C₁-C₄-alkyl, C₁-C₄- alkoxy, hydroxy, C₁-C₄-acyloxy, phenoxy, cyano, nitro, amino,
            and p represents an integer from 0 to 3;
               - a residue of formula SO₂R7 wherein R7 is C₁-C₆-alkyl, C₃-C₆-cycloalkyl, C₂-C₆-alkenyl, aryl and heteroaryl;
               or
            in said formula (II)
            X together with the nitrogen atom to which it is bound and with the **R2** group, forms a nitrogen containing 3-7 membered heterocyclic, monocyclic or polycyclic ring, and **R3** represents hydrogen, C₁-C₄ alkyl, C₁-C₄ acyl, unsubstituted or substituted phenyl with a group selected from halogen, C₁-C₄-alkyl, C₁-C₄-alkoxy, hydroxy, C₁-C₄- acyloxy, phenoxy, cyano, nitro, amino;
   ii) **R1** is linear or branched C₁-C₅ alkyl, C₃-C₅ cycloalkyl;
   iii) **Ar is**
      - a phenyl group unsubstituted or substituted by one or more groups independently selected from halogen, C₁-C₄-alkyl, C₁-C₄-alkoxy, hydroxy, C₁-C₄-acyloxy, phenoxy, cyano, nitro, amino, C₁-C₄-acylamino, halo-C₁- C₃- alkyl, halo-C₁-C₃-alkoxy, benzoyl, heteroaryl carbonyl, heteroaryl, linear or branched C₁-C₈-alkanesulfonate, linear or branched C₁-C₈-alkanesulfonamides, linear or branched C₁-C₈ alkyl sulfonylmethyl; or
      - a heteroaryl ring selected from pyridine, pyrrole, thiophene, furan, indole.

Among the above compounds, particularly preferred are compounds of said formula (I) or pharmaceutically acceptable salts thereof, wherein:
R is selected from:
   - 2-thiazolyl or 2-oxazolyl, unsubstituted or substituted by a group selected from methyl, tert-butyl or trifluoromethyl group;
   - C(Ra)=N-W wherein W is linear or branched C₁-C₄ alkyl,
   - CORa, SORa or SO₂Ra,
wherein Ra is as defined above; and
**Ar** is selected from:
   3 '-benzoylphenyl, 3 '-(4-chloro-benzoyl)-phenyl, 3 '-(4-methyl-benzoyl)-phenyl, 3 '-acetyl-phenyl, 3'-propionyl-phenyl, 3'-isobutanoyl-phenyl, 4'-isobutyl-phenyl, 4' - trifluoromethanesulfonyloxy-phenyl, 4'-benzenesulfonyloxy-phenyl, 4'-trifluoromethanesulfonylamino-phenyl, 4'-benzenesulfonylamino-phenyl, 4'-benzenesulfonylmethyl-phenyl, 4'-acetoxyphenyl, 4'-propionyloxy-phenyl, 4'-benzoyloxy-phenyl, 4'-acetylamino-phenyl, 4'-propionylamino-phenyl, 4'-benzoylamino-phenyl, 3 '-(furan-2-carbonyl)-phenyl, 3 '-(benzofuran-2-carbonyl)-phenyl, 3 '-(thiophen-2-carbonyl)-phenyl, 3 '-(pyridine-2-carbonyl)-phenyl, 3 '-(thiazole-2-carbonyl)-phenyl, 3'-(oxazole-2-carbonyl)-phenyl, 3'-(2-furyl)-phenyl, 3'-(2-oxazolyl)-phenyl, 3'-(3-isoxazolyl)-phenyl, 3'-(2-benzoxazolyl)-phenyl, 3'-(3- benzoisoxazolyl)-phenyl, 3'-(2-thiazolyl)-phenyl, 3'-(2-pyridyl)-phenyl, 3'-(2- thiophenyl)-phenyl;
   or Ar is a heteroaryl ring selected from pyridine, pyrrole, thiophene, furan or indole. Preferred compounds of formula (I) according to the invention are selected from:
      - 4-{(1R)-1-[(phenylsulfonyl)amino]ethyl}phenyl trifluoromethanesulfonate;
      - N-[(1R)-1-(3-benzoylphenyl)ethyl]benzenesulfonamide;
      - 4-{(1R)-1-[(pyridine-3-ylsulfonyl)amino]ethyl}phenyltrifluoromethanesulfonate;
      - N-[(1R)-1-(3-benzoylphenyl)ethyl]methanesulfonamide;
      - N-{(1R)-1-[3-(2-furoyl)phenyl]ethyl}thiophene-2-sulfonamide;
      - N-{(1R)-1-[3-(2-furoyl)phenyl]ethyl}methanesulfonamide;
      - 4-{(1R)-1-[(thien-2-ylsulfonyl)amino]ethyl}phenyl trifluoromethanesulfonate;
      - N-[(1R)-1-(3-benzoylphenyl)ethyl]thiophene-2-sulfonamide;
      - N-[(1R)-1-(3-benzoylphenyl)ethyl]-3-pyrrolidin-1-ylpropane-1-sulfonamide;
      - methyl 5-({[(1R)-1-(3-benzoylphenyl)ethyl]amino}sulfonyl)-2-furoate;
      - 5-({[(1R)-1-(3-benzoylphenyl)ethyl]amino}sulfonyl)-2-furoic acid;
      - 4-{(1R)-2-methyl-1-[(methylsulfonyl)amino]propyl}phenyltrifluoromethanesulfonate;
      - N-((1R)-1-{4-[1-methyl-1-(phenylsulfonyl)ethyl]phenyl}ethyl)methanesulfonamide;
      - 4-[(1R)-1-(isobutyrylamino)ethyl]phenyltrifluoromethanesulfonate;
      - 4-{[(1R)-1-(pyridine-3-ylcarbonyl)amino]ethyl]}phenyltrifluoromethanesulfonate;
      - N-[(1R)-1-(3-benzoylphenyl)ethyl]benzamide;
      - N-[(1R)-1-(3-benzoylphenyl)ethyl]-2-furamide;
      - N-[(1R)-1-(3-benzoylphenyl)ethyl]cyclobutanecarboxamide;
      - N-[(1R)-1-(4-trifluoromethanesulfonyloxy)phenylethyl]-4-piperidin-1-yl butanamide (DF2593Y) ;
      - 4-{(1R)-1-[(4-pyrrolidin-1-ylbutanoyl)amino]ethyl]}phenyl trifluoromethanesulfonate;
      - 3-1(1R)-1-[4-(4-trifluoromethyl-1,3-thiazol-2-yl)amino]ethyl}phenyl) (phenyl)methanone;
      - R(-)-2-[(4'-trifluoromethanesulfonyloxy)phenyl]-N-[3-(N'-pirrolidinyl)propyl] propionamide (DF2297X) or its chloride salt (DF2297A); and
      - 5-[(1*R*)-1-(4-{[4-(trifluoromethyl)-1,3-thiazol-2-yl]amino}phenyl)ethyl]tetrazol-2-ide (DF3966Y) or its sodium salt (DF3966A).

Particularly preferred are compounds of formula (I) according to the invention are selected from N-[(1R)-1-(4-trifluoromethanesulfonyloxy)phenylethyl]-4-piperidin-1-yl butanamide (DF2593Y) and pharmaceutically acceptable salts thereof, preferably its chloride salt (DF2593A), R(-)-2-[(4'-trifluoromethanesulfonyloxy)phenyl]-N-[3-(N'-pirrolidinyl)propyl] propionamide (DF2297X) or its chloride salt (DF2297A), and 5-[(1*R*)-1-(4-{[4-(trifluoromethyl)-1,3-thiazol-2-yl]amino}phenyl)ethyl]tetrazol-2-ide (DF3966Y) or its sodium salt (DF3966A).

Compounds of formula (I) are disclosed in WO2007/060215, which also discloses their method of synthesis, their activity as C5aR inhibitors as well as their use in the treatment of diseases dependent on activation of C5a pathway.

Preferred C5aR1 inhibitors according to the invention are Avacopan, PMX-53, W54011, Avdoralimab, MOR-044254, PMX-205, DF2593A, DF2297A, DF2427 and DF3966A.

The C5aR1 antagonists or allosteric inhibitors of the present invention may form stable pharmaceutically acceptable salts with a pharmaceutically acceptable organic or inorganic acid or base, and in such cases administration of the C5aR1 inhibitor compound as a salt may be appropriate.

Examples of acid addition salts include: acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include: ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Nontoxic, physiologically-acceptable salts are preferred.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes stereoisomers, isotope-labelled, for example deuterated, derivatives and enantiomers of the C5aR1 inhibitors described above.

Certain compounds may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as mixtures of such isomers in any ratio.

Optic isomers can be obtained by resolution of the racemic mixtures according to conventional processes, e.g., by formation of diastereomeric salts, by treatment with an optically active acid or base or enzymatically. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

The C5aR1 inhibitors of the present invention may be in amorphous or crystalline form, including any polymorphic form.

Typically, the C5aR1 inhibitor for use according to the invention is administered in the form of a pharmaceutical composition.

Accordingly, a second aspect of the present invention relates to a pharmaceutical composition comprising a C5aR1 inhibitor, as previously defined, and at least one pharmaceutically acceptable excipient, for use in the prevention or treatment of a hypersensitivity reaction (HSR) to a taxane in an individual, as described above.

Preferably, the pharmaceutical composition of the present invention is prepared in suitable dosage forms comprising an effective amount of the C5aR1 inhibitor, a pharmaceutically acceptable salt therof or a prodrug thereof, and at least one pharmaceutically acceptable excipient.

The administration of the pharmaceutical composition of the present invention to a patient is in accordance with known methods and may comprise oral administration, parenteral administration, preferably selected from intravenous, intraperitoneal, intramuscular, intraarterial, subcutaneous administration, topical administration, buccal administration or rectal (suppository) administration.

In the present description and in the following claims, the wording "effective amount" means a dosage of a drug compound sufficient to significantly achieve the desired clinical response.

The dosage and treatment regimens of the C5aR1 inhibitor for use according to the invention for a particular patient will vary depending on a number of factors that are within the knowledge and expertise of the skilled person including, for example, the inhibitory concentration (IC₅₀) and half life of the specific inhibitor employed, the formulation and route of administration used, the age, body weight, general health status, sex, and diet of the patient.

As described herein, the pharmaceutical composition of the present invention comprises a C5aR1 inhibitor together with a pharmaceutically acceptable excipient, which, as used herein, includes any and all solvents, diluents or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions.

Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any limitation, any material, that is not a drug, suitable for preparing a pharmaceutical composition to be administered to a living being.

The dosage forms can also contain other traditional ingredients such as: preservatives, stabilizers, surfactants, buffers, osmotic regulators, emulsifiers, sweeteners, colorants, flavourings and the like.

The dosage forms of the pharmaceutical composition of the present invention can be prepared by techniques that are familiar to a pharmaceutical chemist, and comprise mixing, granulation, compression, dissolution, sterilization and the like.

Preferably, in the method according to the invention, said C5aR1 is administered in form of a pharmaceutical composition, as above defined.

The invention will be further described in the following examples, which do not limit the scope of the invention defined in the claims.

### EXPERIMENTAL SECTION

### Examples 1-3

BIAcore technology was used to analyze the interaction between Paclitaxel and complement component 5a receptor 1 (C5aR1) (Example 1), complement component 5a receptor 2 (C5AR2) (Example 2) or complement component 5a (Example 3).

The BIAcore systems exploit the phenomenon of Surface Plasmon Resonance (SPR) to monitor interactions between molecules in real time. Briefly, SPR phenomenon occurs in thin conducting films at an interface between media of different refractive index. In BIAcore systems, the media are the glass of the sensorchip and the sample solution, and the conducting film is a thin layer of gold on the sensorchip surface. BIAcore monitors the interaction between two molecules, one attached to the sensor surface, called ligand, and the other one free in solution, and called analyte.

### Example 1

### Binding of Paclitaxel to C5aR1

### a) Immobilization of C5aR1 on a sensorchip CM5

An anti-c-Myc antibody (9E10, sc-40, Santa Cruz Technology) was immobilized on a sensorchip CM5 (#29149604, Cytiva). In details, the antibody was diluted at a concentration of 50 µg/ml in 10 mM acetate buffer at pH 4.5, and injected over the surface of the sensorchip. In details, the dextran matrix on the sensorchip surface was first activated with a mixture of 1-ethyl-3-carbodiimide (EDC) and N-hydroxysuccinimide (NHS) to give reactive succinimide esters. Anti-Myc antibodies were then passed over the surface and the esters reacted spontaneously with uncharged amino groups to covalently link the ligand to the dextran. Then, the surface was deactivated by ethanolamine-HCl.

Covalent immobilization of anti c-Myc antibody by amine-coupling chemistry resulted in stable attachment measured as 2782 resonance units (RU) of ligand to the surface. Once the antibody was immobilized, the receptor was captured on the surface of the sensorchip. This procedure allowed the immobilization of the ligand of interest in an oriented way. In particular, C5AR1-cMyc (TP303784, OriGene) at a concentration of 20 µg/ml in Running Buffer (50mM HEPES buffer, pH 7.0 containing 0.01% CHS, 0.1 % CHAPS, and 0.33 mM of lipids) was injected onto the sensorchip prepared above with a flow rate of 5 µl/min.

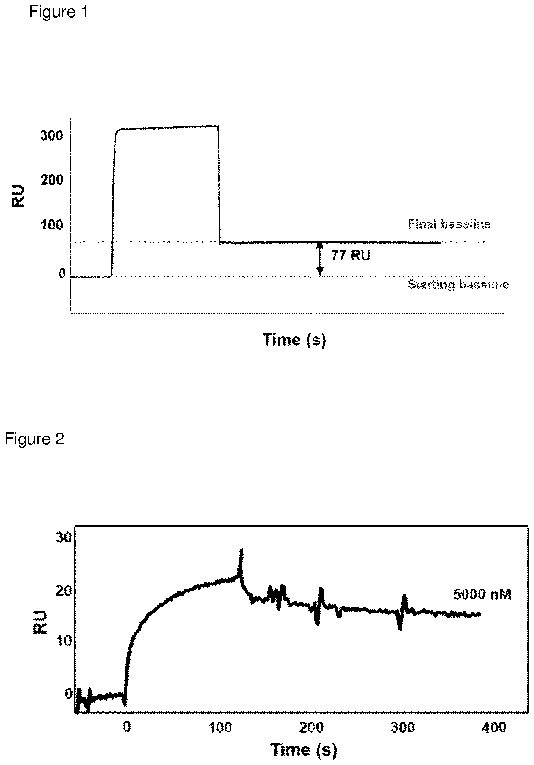

As can be shown in Figure 1, the process results in stable attachment of 77 RU of C5aR1-cMyc to the surface.

### b) Binding of C5a to immobilized C5aR1

To demonstrate that the receptor was immobilized in the correct conformation state, we analyzed the binding of C5a, the natural ligand of the receptor. In details, 5µM C5a (A144, Complement Technologies) in Running Buffer was injected on C5aR1 surface of sensorchip prepared in Example 1a).

As shown in Fig. 2, C5a can bind to the C5aR1 receptor, confirming the correct conformational state of the oriented-immobilized receptor.

### c) Binding of Paclitaxel to immobilized C5aR1

To first evaluate a direct binding of Paclitaxel and C5aR1, the compound was injected on the C5aR1 surface of the sensorchip prepared in Example 1a) at a concentration of 300 µM in Running Buffer.

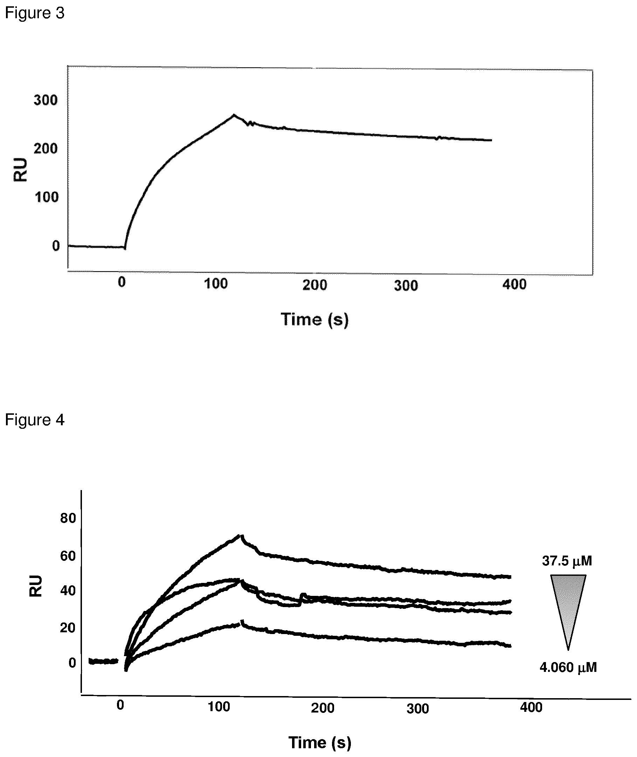

As shown in Fig. 3, Paclitaxel binds the receptor with 300 RU at the concentration tested. In order to determine the affinity value for the interaction, Paclitaxel was diluted in Running Buffer at different concentrations (from 4.06 to 37.5 µM), injected on the sensorchip for 4 minutes and allowed to dissociate for 5 minutes, flowing Running Buffer without Paclitaxel. In this way, the kinetic constant was evaluated as a function of time. As shown in Fig.4, the binding of Paclitaxel to the receptor is dose-dependent with a Kd value of 670 nM.

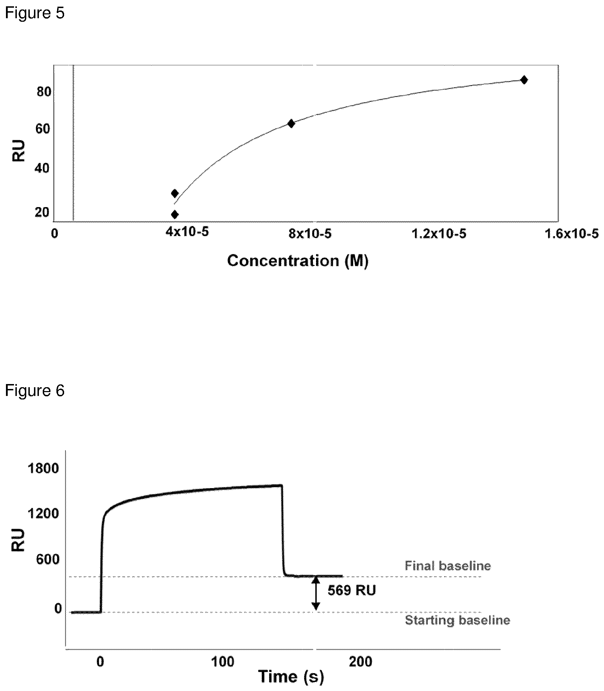

Also, the affinity value at equilibrium of Paclitaxel/Myc-C5aR1 interaction was determined by measuring the steady-state binding levels, through a Scatchard analysis of the RU values at equilibrium. BIAcore provided an affinity constant of 525 nM, as a function of the analyte concentration in solution, as shown in Fig. 5.

### Example 2

### Biding of Paclitaxel to C5aR2

### a) Immobilization of C5aR2 on a sensorchip CM5

A policlonal anti-His antibody (ab137839, Abcam) was immobilized on a sensorchip CM5 (#29149604, Cytiva). In details, the antibody was diluted at a concentration of 50 µg/ml in 10 mM acetate buffer at pH 4.0, and injected over the surface of the sensorchip. Activation and deactivation of sensorchip were performed as described in Example 1a). The covalent immobilization of anti-His antibodies by amine-coupling chemistry resulted in stable attachment of 2510 RU of ligand to the surface. Once the antibody was immobilized, the receptor was captured in an oriented way on the surface of the sensorchip. In particular, C5AR2-His (CSB-CF868390HU, CUSABIO) at a concentration of 100 µg/ml was injected onto the sensorchip with a flow rate of 5 microl/min in Running Buffer.

As can be shown in Fig.6, the process results in the stable attachment of 569 RU of C5aR2 to the surface.

### b) Binding of C5a to immobilized C5aR2

Again, to demonstrate that the receptor was immobilized in the correct conformation state, we analyzed the binding of C5a, the natural ligand of the receptor. 5 µM C5a (A144, Complement Technologies) was injected on C5AR2 surface of the sensorchip prepared in Example 2a).

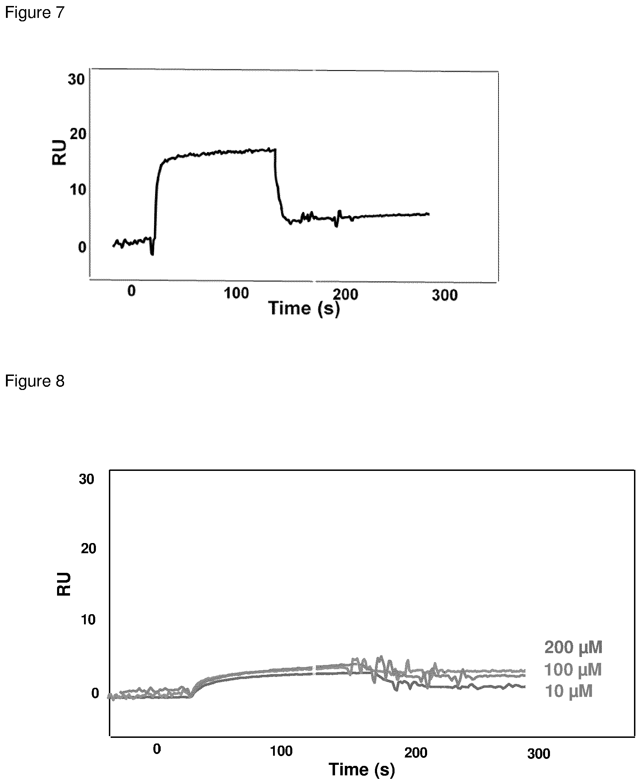

As shown in Fig. 7, C5a is able to bind to the C5aR2 receptor immobilised on the sensorchip, confirming that C5aR2 was immobilized in the right conformation.

### c) Binding of Paclitaxel to immobilized C5aR2

Paclitaxel was injected on the C5aR2 surface of the sensorchip prepared in Example 2a) at different concentrations (10, 100 and 200 microM) in Running Buffer, and the amount of obtained RU was evaluated.

As shown in Fig. 8, Paclitaxel is not able to bind to C5aR2 at any of the concentrations tested.

### Example 3

### Binding of Paclitaxel to C5a

### a) Immobilization of C5a on a sensorchip CM5

C5a was immobilized on a sensorchip (#29149604, Cytiva). In details, 20 microg/ml of recombinant protein C5a was diluted in 10 mM acetate buffer at pH 4.5 and injected over the surface of the sensorchip. Activation and deactivation of sensorchip were performed as described in Example 1a).

The covalent immobilization of C5a by amine-coupling chemistry resulted in stable attachment of 1330 RU of C5a to the surface.

### b) Binding of Paclitaxel to immobilized C5a

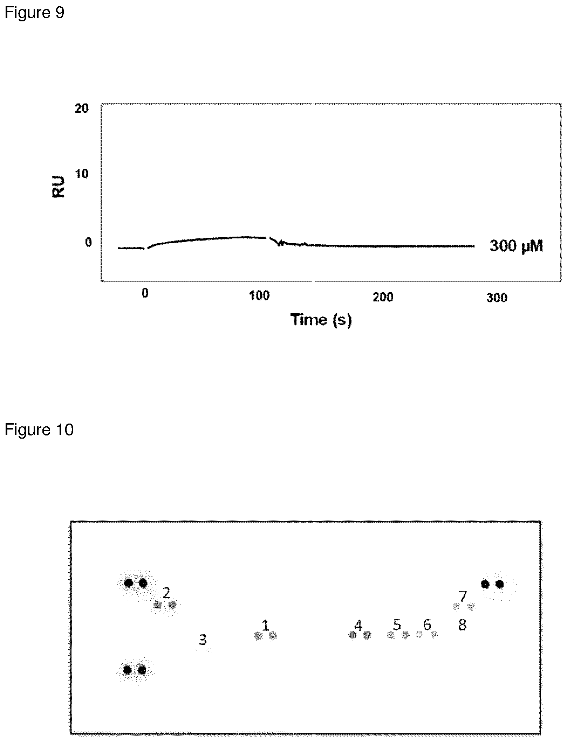

Paclitaxel was injected on the C5A surface of the sensorchip prepared in Example 2a) at at a concentration of 300 microM in Running Buffer.RU can be detected by BIAcore. As shown in Fig. 9, no RU can be detected by BIAcore, thus demonstrating that Paclitaxel is not able to bind to C5a at the high concentration tested.

### Example 4

RAW 264.7 (ATTC TIB-71) mouse macrophages were cultured following manufacturer's instructions using DMEM medium (Euroclone, MI, Italy) supplemented with 10% FBS (non-heat inactivated) (ATCC, USA) at 37 °C, in a humidified 95% air-5% CO₂ atmosphere and then were seeded at 1*104 cells/cm².

After 24 hours, cells were treated with C5a (diluted in medium) (10 nM, R&D, USA), Paclitaxel (diluted in medium) (10 nM, Sigma, USA) in the absence and in the presence of the C5a inhibitor DF3966A (10 µM), and anti C5aR Ab (BioLegend, CA, USA; 50nM) or Avacopan (10 µM) for 24 hours, then the media was replaced with culture media (5 ml) for 24h and then collected.

Conditioned media were centrifugated to remove particulates and assayed immediately. Sample amount was adjusted as suggested (500 µl). The reagents were prepared following manufacturer's protocols (R&D, USA). Briefly, the membranes were incubated with Array Buffer 6 for 1 hour on a rocking platform shaker. The samples were prepared by adding up to 1 ml of Array Buffer 4 in two separated tubes and then 15 microl of reconstituted mouse cytokine detection antibody cocktail (> 40 mouse cytokines) to each prepared sample and incubated 1 hour. Then, the Array Buffer 6 was aspirated and replaced with the sample/antibody mixtures and incubated at 4° C overnight. The following day, membranes were washed 3 times and Streptavidin-HRP (1:2000) was incubated for 30 minutes at room temperature on a rocking platform shaker. The membranes were washed again, and 1 ml of prepared Chemi Reagent mix was placed onto each membrane. Multiple exposure times were acquired using UVITEC digital analyzer (Alliance, Cambridge, UK).

The positive signals seen in the developed membranes can be identified by placing the transparency overlay template on the array image and aligning it with pairs of reference spots in the three corners of each array (see Fig. 10). Reference spots are included to demonstrate that the array was incubated with Streptavidin-HRP during the assay procedure. Pixel densities (average signals of pair of duplicates which represent each cytokine) were analyzed by ImageJ and the average background was subtracted from each spot.

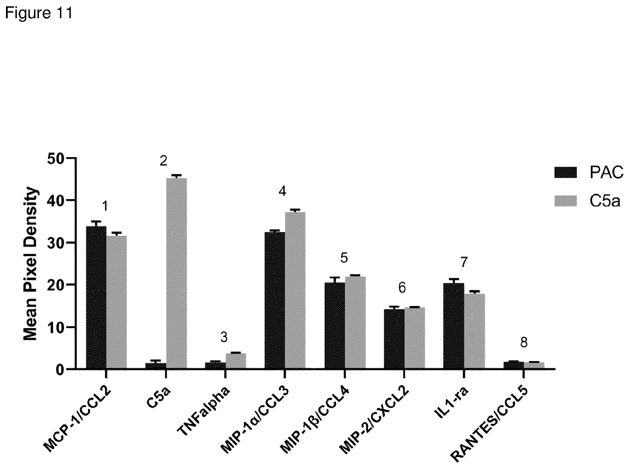

In Figure 11, the proteome profiling of the cytokines released from RAW 264.7 treated with Paclitaxel (PAC) or C5a for 24h is shown. It overlaps for all the analysed cytokines, a part for C5a which is released only by RAW 264.7 cells when stimulated by C5a, and not Paclitaxel. Furthermore, the induction of cytokines release by C5a and Paclitaxel is inhibited by DF3966A, C5aR Ab, and Avacopan, confirming an overlap in the activation of the C5aR1 receptor by the two molecules.

### Example 5

F11 hybridoma cells (ECACC 08062601), chosen as an alternative model of DRG neurons were cultured in DMEM medium (Euroclone, MI, Italy) supplemented with 10% FBS (Sigma-Aldrich St. Louis, CO, USA), 1% penicillin/streptomycin (Euroclone) and 1% glutamine (Euroclone) at 37°C, in a humidified 95% air-5% CO2 atmosphere. For the experiments, cells were used at 18th passage and seeded at 1 x 104 cells/cm2.

After 24h, cells were differentiated with rat NGF (rNGF) (Sigma). rNGF was dissolved in DMEM with 1% penicillin/streptomycin and 1% glutamine (FBS free) at the final concentration 50 ng/ml. The medium was replaced every 3 days until complete differentiation, which happened after 7 days.

Following neuronal differentiation, neurons were treated with paclitaxel (Sigma-Aldrich; 10nM final concentration) or C5a (R&D Systems, Inc. MN, USA; 50nM final concentration) for 20h.

### RT-PCR

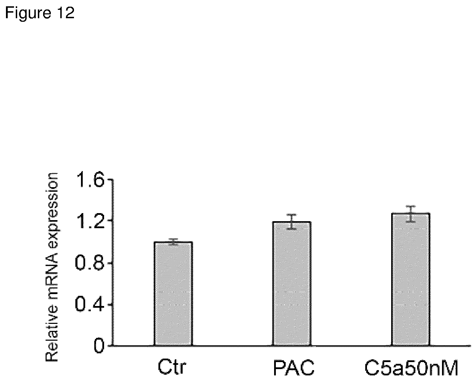

For gene expression analysis, the following protocol was used: total RNA was extracted by Trizol reagent, according to the manufacturer's instructions. The total RNA concentration was determined spectrophotometrically in RNAase-free water, and 1 µg aliquots of total RNA were reverse-transcripted into cDNA using ProtoScript First Strand cDNA Synthesis Kit (NEB). RT-PCR was carried out on ABI 7300HT sequence detection system (ABI), in a total volume of 20 ml containing EagleTaq Universal Master Mix (Roque), DEPC water, 4 microl of cDNA, and the following the Prime Time qPCR Assay for IL-8 Mm04207460m1 (Applied Biosystem, USA). The reference gene GADPH ID Mm04207460g1 (Applied Biosystem, USA) was used as an internal control to normalize the expression of target genes. RT-PCR protocol was as follows: a pre-heating step for 3 minutes at 95 °C, 40 cycles at 95 °C for 10 seconds and 60° for 30 seconds, and last end-step at 65 °C for 10 seconds. Relative expression levels were calculated for each sample after normalization against reference gene, using the ΔΔCt method for comparing relative fold expression differences, as previously described [Livak et al., 2001 DOI: 10.1006/meth.2001.1262]. In Fig. 12 the results of Real Time PCR analysis for IL-8 relative mRNA expression in F11 cells treated for 20h with 10 nM Paclitaxel (PAC) or 50nM C5a is shown. In agreement with the results obtained in macrophages, both treatments increase IL-8 expression levels in differentiated F11. C5a increases IL-8 expression levels, sharing the exact molecular mechanism of Paclitaxel.

The results obtained in these Examples demonstrate that Paclitaxel is able to bind C5aR1, to activate the same signaling pathways of C5a in RAW 264 mouse macrophages and to share the same molecular mechanism of C5a in F11 cells.

### Example 6

### C5aR1 inhibition counteracts paclitaxel- or C5a- induced cytokine release in macrophages

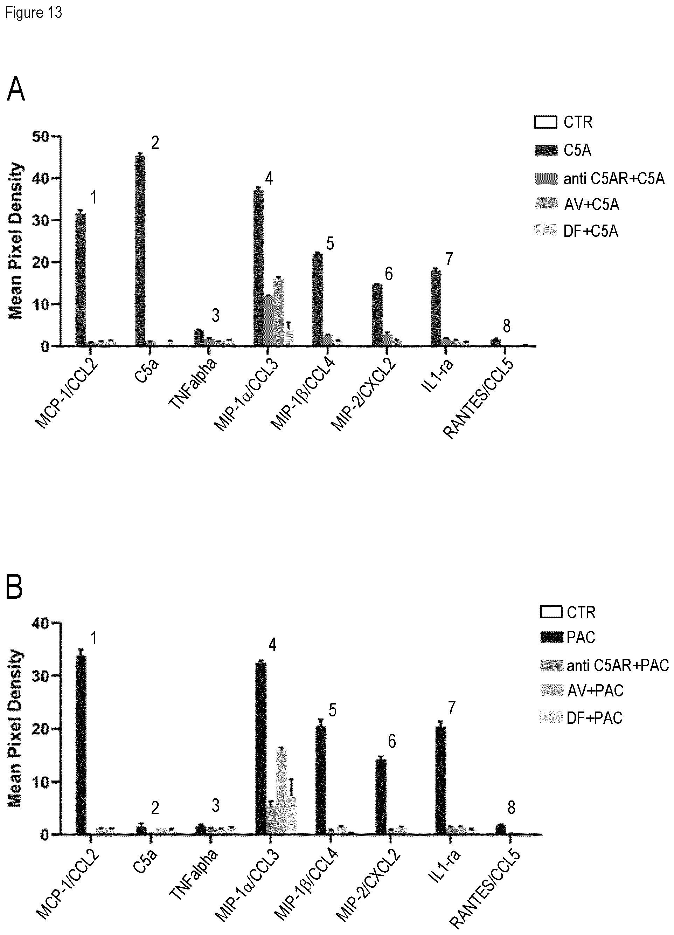

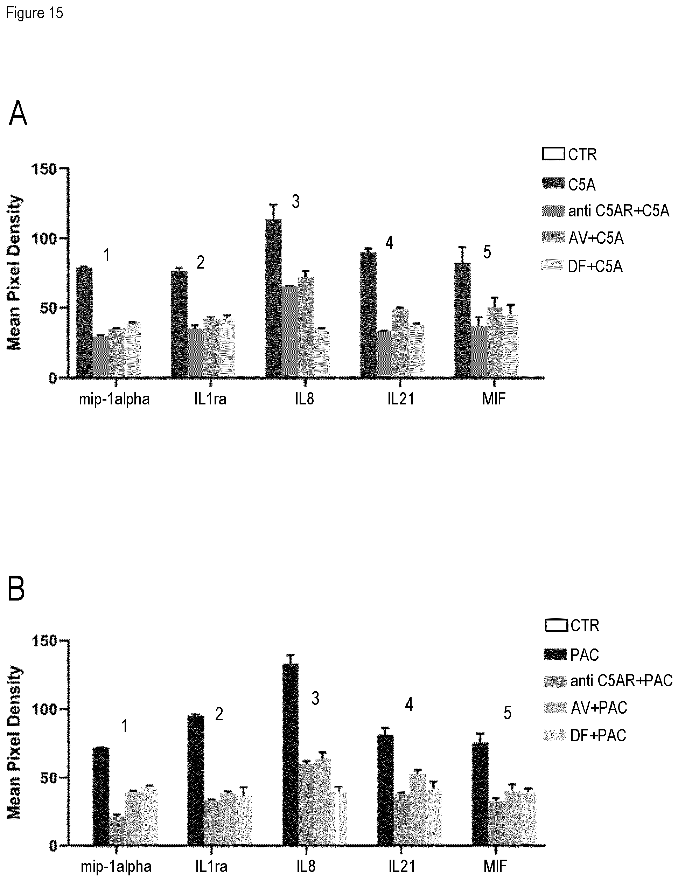

Mouse (RAW 264.7) and human (THP-1) macrophages have been used to demonstrate that C5aR1 mediates paclitaxel-induced HSRs. In details, the profiling of cytokines released (> 40 cytokines) from RAW 264.7 and human macrophages challenged with paclitaxel (Sigma-Aldrich; 10nM) or C5a (R&D Systems, Inc. MN, USA; 10nM) for 24h using a proteome profiler assay. Upon both treatments, murine and human proteome profiles resembled that of typical anaphylaxis and overlapped for all cytokines analyzed, excluding C5a, that was released only by C5a-stimulated cells (Figure 13 and 15). The experimental assay is explained in example 4.

For the human macrophages, human XL Cytokine Array kit #Ary022B was used, while for murine macrophages mouse cytokine Array Panel A #Ary006 was used (R&D systems, USA).

In paclitaxel- and C5a-treated RAW 264.7, the levels of monocyte chemoattractant protein-1 (MCP-1) resulted significantly increased compared to the control group, as well as those of macrophage inflammatory protein (MIP)-1α and MIP-1β and of MIP-2 and IL1Ra (Figure 13A-B).

The release of these cytokines was counteracted by inhibition of C5aR1 mediated by Avacopan (Selleckchem, USA), by an anti-C5aR1 antibody (BioLegend, CA, USA) or by DF3966A (Figures 13A-B). The analysis on human macrophages confirmed the data obtained with murine macrophages as the profile of cytokine released upon C5a overlapped with that obtained upon paclitaxel stimulus, and also in these experimental conditions Avacopan (Selleckchem, USA), an anti-C5aR1 antibody (BioLegend, CA, USA) or DF3966A effectively counteracted the C5a- and paclitaxel-induced cytokine release (Figures 15A-B).

### Example 7

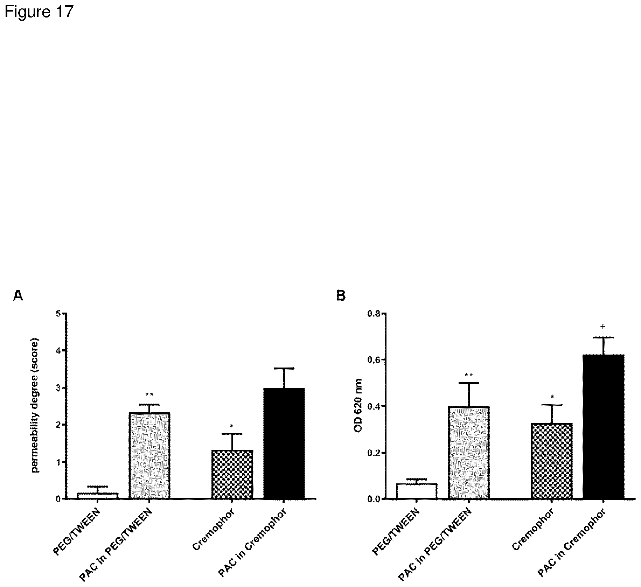

Balb/c mice were thus divided in 4 groups and treated with paclitaxel in Cremophor EL (20 mg/kg and 0.1mL/Kg, iv), paclitaxel in DMSO/PEG/TWEEN (20 mg/kg and 0.1mL/Kg, iv), Cremophor EL alone (0.1mL/Kg, iv) or DMSO/PEG/TWEEN alone (0.1mL/Kg, iv). Mice receiving paclitaxel in Cremophor EL or in DMSO/PEG/TWEEN had acute HSRs that were more serious displaying greater vascular leakage (hyper-permeability) and plasma extravasation compared to mice treated with both vehicles alone (Figures 17A and B), thus confirming the crucial role of paclitaxel *per se* in inducing HSRs.

### Example 8

### C5aR1 inhibition prevents paclitaxel-induced HSRs in vivo

Having identified paclitaxel as the key factor responsible for the cytokines release, typical of HSRs induced by the chemotherapy treatment, we then investigated the potential effects of the inhibition of C5aR1 as preventive treatment.

Paclitaxel (Selleckchem, USA, cod. S1150) was intravenousely administrated at 20 mg/kg (0.1mL/Kg) dissolved in Cremophor EL (polyoxyl 35 castor oil, Sigma-Aldrich, cod. 238470). DF3966A (30mg/kg, os) or DF3966A vehicle (saline) (PAC in Crem +DF and PAC in Cremophor +Veh DF, respectively) were administrated 3h before paclitaxel, while the vehicle used for Avacopan (PEG-400/solutol-HS15 70:30, 5 mL/kg⁵⁰) (PAC in Cremophor +Veh AVA) or Avacopan (30 mg/kg, os) (PAC in Crem +AVA) were administrated 1h before paclitaxel, according to their pharmacokinetic profile. Cremophor alone (Cremophor) was used as a control.

### a) Assessment of Vascular Leakage

Evans Blue (EB, 0.8%/0.3mL/iv) was used to visualize the ear blue colour and quantify the extent of vascular leakage in ears, so as to assess the skin manifestation of paclitaxel injection induced HSRs. 30 minutes after paclitaxel administration mice received EB; the degree of ear colouring was assessed by a score from 0 - 5 where "0" did not represent the visible blue area and "1 to 5" indicated the ratio of the visible blue area.

### b) Plasma extravasation

About 40 minutes following paclitaxel administration, the animal was sacrificed, the ears were removed, shredded and stored in 2mL of formaldehyde overnight at room temperature. The day after the ears were centrifuged and the supernatant was taken and analysed by the spectrophotometer (620 nm O.D.).

### c) ELISA assay

Intracardiac injection was done to obtain blood in tubes and leaving it undisturbed at room temperature. Serum was separated from blood cells 30 minutes later by centrifugation (2000 x g for 10 min, at 4°C). Aliquots were prepared and stored at -20°C. ELISA kit was used to evaluate the following marker: histamine and SC5b-9 (Mybiosource, San Diego, USA Cat.No: MBS9361379, MBS725193).

### d) Results

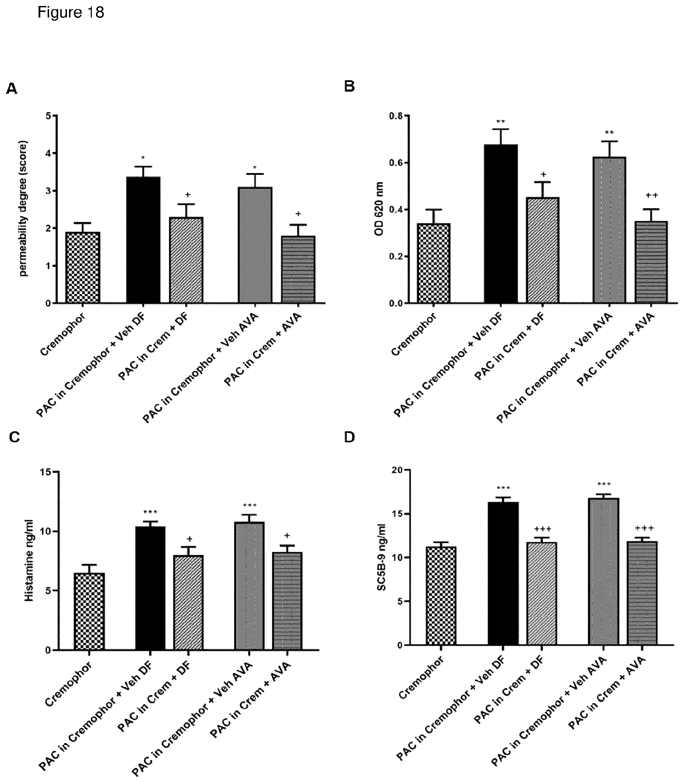

Pharmacological inhibition of C5aR1 by both treatments effectively counteracted the onset of HSRs abolishing paclitaxel-induced increase of vascular leakage and plasma extravasation (Figures 18A and B, respectively). Moreover, pre-treatment with DF3966A or Avacopan significatively reduced the increment of serum concentration of both histamine and complement 5b-9 (SC5b-9) (Figures 18C and D, respectively) in paclitaxel-treated mice, demonstrating that inhibition of C5aR1 is an effective pharmacological strategy for the prevention of paclitaxel-induced HSRs.

## Claims

1. A C5aR1 inhibitor for use in the prevention or treatment of a hypersensitivity reaction (HSR) to a taxane in an individual.

2. A C5aR1 inhibitor for use as claimed in claim 1, wherein said taxane is selected from paclitaxel, N*ab*^{™}-paclitaxel, docetaxel and cabazitaxel.

3. A C5aR1 inhibitor for use as claimed in claim 1 or 2, wherein said C5aR1 inhibitor is for use in the prevention of a hypersensitivity reaction to a taxane in said individual and the inhibitor is administered one or more times prior to each administration of said taxane.

4. A C5aR1 inhibitor for use as claimed in claim 3, wherein said C5aR1 inhibitor is administered one or more times between 30 minutes and 24 hours before administering the taxane to the individual.

5. A C5aR1 inhibitor for use as claimed in claim 3 or 4, wherein said individual is a cancer patient that has had a previous HSR episode to a taxane, has resulted positive in a skin prick test for sensitivity to said taxane and/or has a history of atopy.

6. A C5aR1 inhibitor for use as claimed in claims 3 to 5, wherein it is administered in combination with one or more corticosteroids and/or antihistamines.

7. A C5aR1 inhibitor for use as claimed in claim 1 or 2, wherein said C5aR1 inhibitor is for use in the acute treatment of a hypersensitivity reaction to a taxane in said individual and the inhibitor is administered at the onset of the symptoms of the hypersensitivity reaction.

8. A C5aR1 inhibitor for use as claimed in claims 1 to 7, wherein said C5aR1 inhibitor is selected from C5aR1 competitive antagonists, anti-C5aR1 antibodies able to block the C5a binding sites on the receptor and C5aR1 non-competitive allosteric inhibitors.

9. A C5aR1 inhibitor for use as claimed in claims 1 to 8, which is a C5aR1 antagonists selected from the group consisting of:
- (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide (Avacopan, Vynpenta^{®});
- N-Acetyl-L-phenylalanyl-L-ornithyl-L-prolyl-3-cyclohexyl-D-alanyl-L-tryptophyl-L-arginine-N-5.2-C-1.6-lactam (PMX-53);
- Acetylated phenylalanine-[ornithyl-proline-(D)cyclohexylalanine-tryptophyl-arginine];
- L-Alanyl-L-seryl-glycyl-L-alanyl-L-prolyl-L-alanyl-L-prolyl-glycyl-L-prolyl-L-alanyl-glycyl-L-prolyl-L-leucyl-L-arginyl-L-prolyl-L-methionyl-L-phenylalanine;
- N,N-Bis(1,3-benzodioxol-5-ylmethyl)-N-(1-butyl-2,4-diphenyl-1H-imidazol-5-ylmethyl)amine;
- N-[2-(4-Chlorophenyl)ethyl]-N-(1,4-dioxaspiro[4.5]dec-8-yl)-2-isobutylbenzamide;
- N-[2-(4-Chlorophenyl)ethyl]-N-(4-hydroxycyclohexyl)-1-benzothiophene-3-carboxamide;
- N-[2-(4-Chlorophenyl)ethyl]-N-(4-hydroxycyclohexyl)naphthalene-1-carboxamide;
- 2-(2-Ethyl-6-methylphenyl)-4-methoxy-N-(5-methoxy-2-methylphenyl)-5,6,7,8-tetrahydroquinolin-5-amine;
- 2-(2,6-Diethylphenyl)-N-ethyl-4-methoxy-N-(1-naphthyl)-5,6,7,8-tetrahydroquinolin-5-amine;
- N-[2,6-Dioxohexahydropyrimidin-4(S)-ylcarbonyl]-L-phenylalanyl-L-ornithyl-L-prolyl-5-methyl-L-norleucyl-4-fluoro-L-phenylalanyl-L-phenylalaninamide (JPE-1375; JSM-1375);
- N-(3-Phenylpropionyl)-L-ornithyl-L-prolyl-3-cyclohexyl-D-alanyl-L-tryptophyl-L-arginine N-5.1-C-1.5-lactam (PMX-205);
- N,N'-Bis(4-amino-2-methylquinolin-6-yl)urea (NSC12155);
- N-[4-(Dimethylamino)benzyl]-N-(4-isopropylphenyl)-7-methoxy-1,2,3,4-tetrahydronaphthalene-1-carboxamide hydrochloride (W54011);
- L-Phenylalanyl-L-ornithyl-L-prolyl-3-cyclohexyl-D-alanyl-L-tryptophyl-L-arginine N-5.2-C-1.6-cyclic peptide;
- (4aR, 16aS)-6, 18-Dihydroxy-23(S)-[2(S)-hydroxy-2-[2(R)-hydroxy-6(R)-methyl-5(R)-[2(S)-methylbutyl]tetrahydro-2H-pyran-2-yl]propionamido]-22(S)-isopropyl-7(S),19(R)-dimethyldocosahydro-13H,22H-dipyridazino[6,1-f:6',1'-o][1,4,7,10,13,16]oxapentaazacyclononadecine-5,7,11,17,20,24-hexanone (L-156602).

10. A C5aR1 inhibitor for use as claimed in claims 1 to 8, which is an anti-C5aR1 antibody selected from the group consisting of:
- Avdoralimab (IPH-5401)
- MOR-044254;
- NOX-D20;
- Anti-C5aR1ab-C5-SiRNA;
- m20/70 mIgG2a.1;
- 3C5;
- 7F3.

11. A C5aR1 inhibitor for use as claimed in claims 1 to 8, which is a non-competitive allosteric inhibitor selected from
- (2*R*)-2-[3-(furan-2-carbonyl)phenyl]-*N*-[4-(trifluoromethyl)-1,3-thiazol-2-yl]propenamide (DF2427); and
- compounds having general formula (I) 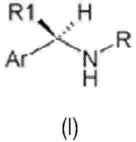 or a pharmaceutically acceptable salt thereof, wherein
i) R is selected from:
- 2 -thiazolyl or 2-oxazolyl, unsubstituted or substituted by a group selected from methyl, tert -butyl or trifluoromethyl group;
- C(Ra)=N-W wherein W is linear or branched C₁-C₄ alkyl,
- CORa, SORa, SO₂Ra, PORa, PO₂Ra, wherein
**Ra** is selected from
- C₁-C₅-alkyl, C₃-C₆-cycloalkyl, C₂-C₅-alkenyl, unsubstituted or substituted phenyl with a group selected from halogen, C₁-C₄-alkyl, C₁-C₄-alkoxy, halo-C₁-C₄-alkoxy, hydroxy, C₁-C₄-acyloxy, phenoxy, cyano, nitro, amino;
- a heteroaryl group selected from pyridine, pyrimidine, pyrrole, thiophene, furane, indole, thiazole, oxazole, such heteroaryl being unsubstituted or substituted with a group selected from halogen, C₁-C₄-alkyl, C₁-C₄-alkoxy, halo-C₁-C₄-alkoxy, hydroxy, C₁-C₄- acyloxy, phenoxy, cyano, nitro, amino;
- a α or β carboxyalkyl residue consisting of straight or branched C₁- C₆-alkyl, C₃-C₆-cycloalkyl, C₂-C₆-alkenyl, C₁-C₆-phenylalkyl, optionally substituted with a further carboxy (COOH) group;
- an ω-aminoalkylamino group of formula (II): 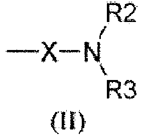 wherein in said formula (II)
X is:
- linear or branched C₁-C₆ alkylene, C₄-C₆ alkenylene, C₄-C₆ alkynylene, optionally substituted by: a) a CO₂R4 group, wherein R4 represents hydrogen or a linear or branched C₁-C₆ alkyl group or a linear or branched C₂-C₆ alkenyl group, or b) by a CONHR5 group wherein R5 represents hydrogen, linear or branched C₂- C₆ alkyl or an OR4 group, R4 being defined as above;
- a (CH₂)ₘ-B-(CH₂)ₙ, group, optionally substituted by a CO₂R4 or CONHR5 group, as defined above, wherein a) B is an oxygen, or sulfur atom, or nitrogen atom optionally substituted by a C₁-C₄ alkyl group, m is zero or an integer from 2 to 3 and n is an integer from 2 to 3, or b) B is a CO, SO or CONH group, m is an integer from 1 to 3 and n is an integer from 2 to 3;
**R2** and **R3** are independently hydrogen, linear or branched C₁-C₆ alkyl, optionally interrupted by an oxygen or sulfur atom, a C₃-C₇ cycloalkyl, C₃-C₆ alkenyl, C₃-C₆-alkynyl, aryl-C₁-C₃-alkyl, hydroxy-C₂-C₃-alkyl group; or
**R2** and **R3** together with the N atom to which they are bound, form a 3-7 membered nitrogen heterocyclic ring of formula (III) 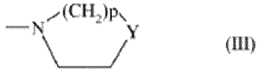 wherein
Y represents:
- a single bond, CH₂, O, S, or a N-R6 group, where R6 represents hydrogen, C₁-C₄ alkyl, C₁-C₄ acyl, unsubstituted or substituted phenyl with a group selected from halogen, C₁-C₄-alkyl, C₁-C₄- alkoxy, hydroxy, C₁-C₄-acyloxy, phenoxy, cyano, nitro, amino,
and p represents an integer from 0 to 3;
- a residue of formula SO₂R7 wherein R7 is C₁-C₆-alkyl, C₃-C₆-cycloalkyl, C₂-C₆-alkenyl, aryl and heteroaryl;
or
in said formula (II)
X together with the nitrogen atom to which it is bound and with the **R2** group, forms a nitrogen containing 3-7 membered heterocyclic, monocyclic or polycyclic ring, and **R3** represents hydrogen, C₁-C₄ alkyl, C₁-C₄ acyl, unsubstituted or substituted phenyl with a group selected from halogen, C₁-C₄-alkyl, C₁-C₄-alkoxy, hydroxy, C₁-C₄- acyloxy, phenoxy, cyano, nitro, amino;
ii) **R1** is linear or branched C₁-C₅ alkyl, C₃-C₅ cycloalkyl;
iii) **Ar** is
- a phenyl group unsubstituted or substituted by one or more groups independently selected from halogen, C₁-C₄-alkyl, C₁-C₄-alkoxy, hydroxy, C₁-C₄-acyloxy, phenoxy, cyano, nitro, amino, C₁-C₄-acylamino, halo-C₁- C₃- alkyl, halo-C₁-C₃-alkoxy, benzoyl, heteroaryl carbonyl, heteroaryl, linear or branched C₁-C₈-alkanesulfonate, linear or branched C₁-C₈-alkanesulfonamides, linear or branched C₁-C₈ alkyl sulfonylmethyl; or
- a heteroaryl ring selected from pyridine, pyrrole, thiophene, furan, indole.
Among the above compounds, particularly preferred are compounds of said formula (I) or pharmaceutically acceptable salts thereof, wherein:
R is selected from:
- 2-thiazolyl or 2-oxazolyl, unsubstituted or substituted by a group selected from methyl, tert-butyl or trifluoromethyl group;
- C(Ra)=N-W wherein W is linear or branched C₁-C₄ alkyl,
- CORa, SORa or SO₂Ra,
wherein Ra is as defined above; and
**Ar** is selected from:
3 '-benzoylphenyl, 3 '-(4-chloro-benzoyl)-phenyl, 3 '-(4-methyl-benzoyl)-phenyl, 3 '-acetyl-phenyl, 3'-propionyl-phenyl, 3'-isobutanoyl-phenyl, 4'-isobutyl-phenyl, 4' - trifluoromethanesulfonyloxy-phenyl, 4'-benzenesulfonyloxy-phenyl, 4'-trifluoromethanesulfonylamino-phenyl, 4'-benzenesulfonylamino-phenyl, 4'-benzenesulfonylmethyl-phenyl, 4'-acetoxyphenyl, 4'-propionyloxy-phenyl, 4'-benzoyloxy-phenyl, 4'-acetylamino-phenyl, 4'-propionylamino-phenyl, 4'-benzoylamino-phenyl, 3 '-(furan-2-carbonyl)-phenyl, 3 '-(benzofuran-2-carbonyl)-phenyl, 3 '-(thiophen-2-carbonyl)-phenyl, 3 '-(pyridine-2-carbonyl)-phenyl, 3 '-(thiazole-2-carbonyl)-phenyl, 3'-(oxazole-2-carbonyl)-phenyl, 3'-(2-furyl)-phenyl, 3'-(2-oxazolyl)-phenyl, 3'-(3-isoxazolyl)-phenyl, 3'-(2-benzoxazolyl)-phenyl, 3'-(3- benzoisoxazolyl)-phenyl, 3'-(2-thiazolyl)-phenyl, 3'-(2-pyridyl)-phenyl, 3'-(2- thiophenyl)-phenyl;
or Ar is a heteroaryl ring selected from pyridine, pyrrole, thiophene, furan or indole.

12. A C5aR1 inhibitor for use as claimed in claims 1 to 8 and 11, wherein said compound is selected from:
- (2R)-2-[3-(furan-2-carbonyl)phenyl]-N-[4-(trifluoromethyl)-1,3-thiazol-2-yl]propenamide (DF2427);
- 4-{(1R)-1-[(phenylsulfonyl)amino]ethyl}phenyl trifluoromethanesulfonate;
- N-[(1R)-1-(3-benzoylphenyl)ethyl]benzenesulfonamide;
- 4-{(1R)-1-[(pyridine-3-ylsulfonyl)amino]ethyl}phenyltrifluoromethanesulfonate;
- N-[(1R)-1-(3-benzoylphenyl)ethyl]methanesulfonamide;
- N-{(1R)-1-[3-(2-furoyl)phenyl]ethyl}thiophene-2-sulfonamide;
- N-{(1R)-1-[3-(2-furoyl)phenyl]ethyl}methanesulfonamide;
- 4-{(1R)-1-[(thien-2-ylsulfonyl)amino]ethyl}phenyl trifluoromethanesulfonate;
- N-[(1R)-1-(3-benzoylphenyl)ethyl]thiophene-2-sulfonamide;
- N-[(1R)-1-(3-benzoylphenyl)ethyl]-3-pyrrolidin-1-ylpropane-1-sulfonamide;
- methyl 5-({[(1R)-1-(3-benzoylphenyl)ethyl]amino}sulfonyl)-2-furoate;
- 5-({[(1R)-1-(3-benzoylphenyl)ethyl]amino}sulfonyl)-2-furoic acid;
- 4-{(1R)-2-methyl-1-[(methylsulfonyl)amino]propyl}phenyltrifluoromethanesulfonate;
- N-((1R)-1-{4-[1-methyl-1-(phenylsulfonyl)ethyl]phenyl}ethyl)methanesulfonamide;
- 4-[(1R)-1-(isobutyrylamino)ethyl]phenyltrifluoromethanesulfonate;
- 4-{[(1 R)-1-(pyridine-3-ylcarbonyl)amino]ethyl]}phenyltrifluoromethanesulfonate;
- N-[(1R)-1-(3-benzoylphenyl)ethyl]benzamide;
- N-[(1R)-1-(3-benzoylphenyl)ethyl]-2-furamide;
- N-[(1R)-1-(3-benzoylphenyl)ethyl]cyclobutanecarboxamide;
- N-[(1R)-1-(4-trifluoromethanesulfonyloxy)phenylethyl]-4-piperidin-1-yl butanamide (DF2593Y) ;
- 4-{(1R)-1-[(4-pyrrolidin-1-ylbutanoyl)amino]ethyl]}phenyl trifluoromethanesulfonate;
- 3-{(1R)-1-[4-(4-trifluoromethyl-1,3-thiazol-2-yl)amino]ethyl}phenyl) (phenyl)methanone;
- R(-)-2-[(4'-trifluoromethanesulfonyloxy)phenyl]-N-[3-(N'-pirrolidinyl)propyl] propionamide (DF2297X) or its chloride salt (DF2297A) ; and
- 5-[(1R)-1-(4-{[4-(trifluoromethyl)-1,3-thiazol-2-yl]amino}phenyl)ethyl]tetrazol-2-ide (DF3966Y) or its sodium salt (DF3966A).

13. A pharmaceutical composition comprising a C5aR1 inhibitor as described in claims 1 to 12 and at least one inert pharmaceutically acceptable excipient, for use in the prevention or treatment of a hypersensitivity reaction (HSR) to a taxane in an individual.

## Patentansprüche

1. C5aR1-Inhibitor zur Verwendung bei der Prävention oder Behandlung einer Überempfindlichkeitsreaktion (HSR) auf ein Taxan bei einem Individuum.

2. C5aR1-Inhibitor zur Verwendung nach Anspruch 1, wobei das Taxan aus Paclitaxel, Nab^{™} -Paclitaxel, Docetaxel und Cabazitaxel ausgewählt ist.

3. C5aR1-Inhibitor zur Verwendung nach Anspruch 1 oder 2, wobei der C5aR1-Inhibitor zur Verwendung bei der Prävention einer Überempfindlichkeitsreaktion auf ein Taxan bei dem Individuum ist und der Inhibitor vor jeder Verabreichung des Taxans einmal oder mehrmals verabreicht wird.

4. C5aR1-Inhibitor zur Verwendung nach Anspruch 3, wobei der C5aR1-Inhibitor einmal oder mehrmals zwischen 30 Minuten und 24 Stunden vor der Verabreichung des Taxans an das Individuum verabreicht wird.

5. C5aR1-Inhibitor zur Verwendung nach Anspruch 3 oder 4, wobei das Individuum ein Krebspatient ist, der eine frühere HSR-Episode gegenüber einem Taxan hatte, in einem Haut-Prick-Test auf Empfindlichkeit gegenüber dem Taxan positiv war und/oder eine Vorgeschichte von Atopie aufweist.

6. C5aR1-Inhibitor zur Verwendung nach den Ansprüchen 3 bis 5, wobei er in Kombination mit einem oder mehreren Corticosteroiden und/oder Antihistaminika verabreicht wird.

7. C5aR1-Inhibitor zur Verwendung nach Anspruch 1 oder 2, wobei der C5aR1-Inhibitor zur Verwendung bei der akuten Behandlung einer Überempfindlichkeitsreaktion auf ein Taxan bei dem Individuum ist und der Inhibitor zu Beginn der Symptome der Überempfindlichkeitsreaktion verabreicht wird.

8. C5aR1-Inhibitor zur Verwendung nach den Ansprüchen 1 bis 7, wobei der C5aR1-Inhibitor aus kompetitiven C5aR1-Antagonisten, Anti-C5aR1-Antikörpern, die in der Lage sind, die C5a-Bindungsstellen am Rezeptor zu blockieren, und nichtkompetitiven allosterischen C5aR1-Inhibitoren ausgewählt ist.

9. C5aR1-Inhibitor zur Verwendung nach den Ansprüchen 1 bis 8, der ein C5aR1-Antagonist ist, ausgewählt aus der Gruppe bestehend aus:
- (2R,3S)-2-[4-(Cyclopentylamino)phenyl]-1-(2-fluor-6-methylbenzoyl)-N-[4-methyl-3-(trifluormethyl)phenyl]piperidin-3-carboxamid (Avacopan, Vynpenta^{®});
- N-Acetyl-L-phenylalanyl-L-ornithyl-L-prolyl-3-cyclohexyl-D-alanyl-L-tryptophyl-L-arginin-N-5,2-C-1,6-lactam (PMX-53);
- Acetyliertes Phenylalanin-[ornithyl-prolin-(D)cyclohexylalanin-tryptophyl-arginin];
- L-Alanyl-L-seryl-glycyl-L-alanyl-L-prolyl-L-alanyl-L-prolyl-glycyl-L-prolyl-L-alanylglycyl-L-prolyl-L-leucyl-L-arginyl-L-prolyl-L-methionyl-L-phenylalanin;
- N,N-Bis(1,3-benzodioxol-5-ylmethyl)-N-(1-butyl-2,4-diphenyl-1H-imidazol-5-ylmethyl)amin;
- N-[2-(4-Chlorphenyl)ethyl]-N-(1,4-dioxaspiro[4,5]dec-8-yl)-2-isobutylbenzamid;
- N-[2-(4-Chlorphenyl)ethyl]-N-(4-hydroxycyclohexyl)-1-benzothiophen-3-carboxamid;
- N-[2-(4-Chlorphenyl)ethyl]-N-(4-hydroxycyclohexyl)naphthalin-1-carboxamid;
- 2-(2-Ethyl-6-methylphenyl)-4-methoxy-N-(5-methoxy-2-methylphenyl)-5,6,7,8-tetrahydrochinolin-5-amin;
- 2-(2,6-Diethylphenyl)-N-ethyl-4-methoxy-N-(1-naphthyl)-5,6,7,8-tetrahydrochinolin-5-amin;
- N-[2,6-Dioxohexahydropyrimidin-4(S)-ylcarbonyl]-L-phenylalanyl-L-ornithyl-L-prolyl-5-methyl-L-norleucyl-4-fluoro-L-phenylalanyl-L-phenylalaninamid (JPE-1375; JSM-1375);
- N-(3-Phenylpropionyl)-L-ornithyl-L-prolyl-3-cyclohexyl-D-alanyl-L-tryptophyl-L-arginin N-5,1-C-1,5-lactam (PMX-205);
- N,N'-Bis(4-amino-2-methylchinolin-6-yl)harnstoff (NSC12155);
- N-[4-(Dimethylamino)benzyl]-N-(4-isopropylphenyl)-7-methoxy-1,2,3,4-tetrahydronaphthalin-1-carboxamid-Hydrochlorid (W54011);
- L-Phenylalanyl-L-ornithyl-L-prolyl-3-cyclohexyl-D-alanyl-L-tryptophyl-L-arginin N-5,2-C-1,6-cyclisches Peptid;
- (4aR,16aS)-6,18-Dihydroxy-23(S)-[2(S)-hydroxy-2-[2(R)-hydroxy-6 (R)-methyl-5(R)-[2(S)-methylbutyl]tetrahydro-2H-pyran-2-yl]propionamido]-22(S)-isopropyl-7(S), 19(R)-dimethyldocosahydro-13H,22H-dipyridazino[6, 1-f:6', 1'-o][1,4,7,10,13,16]oxapentaazacyclonadecin-5,7,11,17,20,24-hexanon (L-156602).

10. C5aR1-Inhibitor zur Verwendung nach den Ansprüchen 1 bis 8, der ein Anti-C5aR1-Antikörper ist, ausgewählt aus der Gruppe bestehend aus:
- Avdoralimab (IPH-5401)
- MOR-044254;
- NOX-020;
- Anti-C5aR1 ab-C5-SiRNA;
- m20/70 mlgG2a.1;
- 3C5;
- 7F3.

11. C5aR1-Inhibitor zur Verwendung nach den Ansprüchen 1 bis 8, der ein nichtkompetitiver allosterischer Inhibitor ist, ausgewählt aus
- (2*R*)-2-[3-(Furan-2-carbonyl)phenyl]-*N*-[4-(Trifluormethyl)-1,3-thiazol-2-yl]propenamid (DF2427); und
- Verbindungen mit der allgemeinen Formel (I) 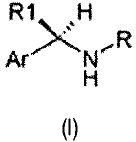
oder einem pharmazeutisch verträglichen Salz davon, wobei:
i) R ausgewählt ist aus:
- 2-Thiazolyl oder 2-Oxazolyl, unsubstituiert oder substituiert mit einer Gruppe, ausgewählt aus Methyl-, tert-Butyl- oder Trifluormethylgruppe;
- C(Ra)=N-W, wobei W lineares oder verzweigtes C₁-C₄-Alkyl ist,
- CORa, SORa, SO₂Ra, PORa, PO₂Ra,
wobei
**Ra** ausgewählt ist aus
- C₁-C₅-Alkyl, C₃-C₆-Cycloalkyl, C₂-C₅-Alkenyl, unsubstituiertem oder substituiertem Phenyl mit einer Gruppe ausgewählt aus Halogen, C₁-C₄-Alkyl, C₁-C₄-Alkoxy, Halogen-C₁-C₄-alkoxy, Hydroxy, C₁-C₄-Acyloxy, Phenoxy, Cyano, Nitro, Amino;
- einer Heteroarylgruppe, ausgewählt aus Pyridin, Pyrimidin, Pyrrol, Thiophen, Furan, Indol, Thiazol, Oxazol, wobei dieses Heteroaryl unsubstituiert oder mit einer Gruppe substituiert ist, ausgewählt aus Halogen, C₁-C₄-Alkyl, C₁-C₄-Alkoxy, Halogen-C₁-C₄-Alkoxy, Hydroxy, C₁-C₄-Acyloxy, Phenoxy, Cyano, Nitro, Amino;
- einem α- oder β-Carboxyalkylrest, bestehend aus geradkettigem oder verzweigtem C₁-C₆-Alkyl, C₃-C₆-Cycloalkyl, C₂-C₆-Alkenyl, C₁-C₆-Phenylalkyl, gegebenenfalls substituiert mit einer weiteren Carboxy (COOH)-Gruppe;
- einer ω-Aminoalkylaminogruppe der Formel (II): 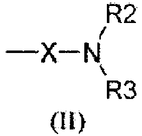 wobei in Formel (II):
X ist:
- lineares oder verzweigtes C₁-C₆-Alkylen, C₄-C₆-Alkenylen, C₄-C₆-Alkinylen, gegebenenfalls substituiert durch: a) eine CO₂ R4-Gruppe, wobei R4 Wasserstoff oder eine lineare oder verzweigte C₁-C₆-Alkylgruppe oder eine lineare oder verzweigte C₂-C₆-Alkenylgruppe darstellt, oder b) durch eine CONHR5-Gruppe, wobei R5 Wasserstoff, lineares oder verzweigtes C₂-C₆-Alkyl oder eine OR4-Gruppe darstellt, wobei R4 wie oben definiert ist;
- eine (CH₂)ₘ-B-(CH₂)ₙ-Gruppe, gegebenenfalls substituiert durch eine CO₂R4-oder CONHR5-Gruppe, wie oben definiert, wobei a) B ein Sauerstoff- oder Schwefelatom oder Stickstoffatom ist, das gegebenenfalls durch eine C₁-C₄-Alkylgruppe substituiert ist, m Null oder eine ganze Zahl von 2 bis 3 ist und n eine ganze Zahl von 2 bis 3 ist, oder b) B eine CO, SO oder CONH-Gruppe ist, m eine ganze Zahl von 1 bis 3 ist und n eine ganze Zahl von 2 bis 3 ist;
**R2** und **R3** unabhängig voneinander Wasserstoff, lineares oder verzweigtes C₁-C₆-Alkyl, gegebenenfalls unterbrochen durch ein Sauerstoff- oder Schwefelatom, eine C₃-C₇-Cycloalkyl-, C₃-C₅-Alkenyl-, C₃-C₅-Alkinyl-, Aryl-C₁-C₃-alkyl-, Hydroxy-C₂-C₃-alkylGruppe; oder
**R2** und **R3** bilden zusammen mit dem N-Atom, an das sie gebunden sind, einen 3-7-gliedrigen Stickstoffheterocyclus der Formel (III) 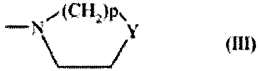
wobei
Y darstellt:
- eine Einfachbindung, CH₂, O, S oder eine N-R6-Gruppe, wobei R6 Wasserstoff, C₁-C₄-Alkyl, C₁-C₄-Acyl, unsubstituiertes oder substituiertes Phenyl mit einer Gruppe ausgewählt aus Halogen, C₁-C₄-Alkyl, C₁-C₄-Alkoxy, Hydroxy, C₁-C₄-Acyloxy, Phenoxy, Cyano, Nitro, Amino darstellt,
und p eine ganze Zahl von 0 bis 3 darstellt;
- ein Rest der Formel SO₂R7, wobei R7 C₁-C₆-Alkyl, C₃-C₆-Cycloalkyl, C₂-C₆-Alkenyl, Aryl und Heteroaryl ist;
oder
in der Formel (II)
bildet **X** zusammen mit dem Stickstoffatom, an das es gebunden ist, und mit der **R2-**Gruppe einen Stickstoff enthaltenden 3-7-gliedrigen heterocyclischen, monocyclischen oder polycyclischen Ring und **R3** Wasserstoff, C₁-C₄-Alkyl, C₁-C₄-Acyl, unsubstituiertes oder substituiertes Phenyl mit einer Gruppe ausgewählt aus Halogen, C₁-C₄-Alkyl, C₁-C₄-Alkoxy, Hydroxy, C₁-C₄-Acyloxy, Phenoxy, Cyano, Nitro, Amino darstellt;
ii) **R1** ist lineares oder verzweigtes C₁-C₅-Alkyl, C₃-C₅-Cycloalkyl;
iii) **Ar** ist
- eine Phenylgruppe, die unsubstituiert oder durch eine oder mehrere Gruppen substituiert ist, die unabhängig ausgewählt sind aus Halogen, C₁-C₄-Alkyl, C₁-C₄-Alkoxy, Hydroxy, C₁-C₄-Acyloxy, Phenoxy, Cyano, Nitro, Amino, C₁-C₄-Acylamino, Halogen-C₁-C₃-alkyl, Halogen-C₁-C₃-alkoxy, Benzoyl, Heteroarylcarbonyl, Heteroaryl, linearem oder verzweigtem C₁-C₈-Alkansulfonat, linearem oder verzweigtem C₁-C₈-Alkansulfonamiden, linearem oder verzweigtem C₁-C₈-Alkylsulfonylmethyl; oder
- ein Heteroarylring, ausgewählt aus Pyridin, Pyrrol, Thiophen, Furan, Indol. Unter den obigen Verbindungen sind besonders bevorzugt Verbindungen der Formel (I) oder pharmazeutisch verträgliche Salze davon, wobei:
R ausgewählt ist aus:
- 2-Thiazolyl oder 2-Oxazolyl, unsubstituiert oder substituiert mit einer Gruppe, ausgewählt aus Methyl-, tert-Butyl- oder Trifluormethylgruppe;
- C(Ra)=N-W, wobei W lineares oder verzweigtes C₁-C₄-Alkyl ist,
- CORa, SORa oder SO₂Ra,
wobei Ra wie oben definiert ist; und
**Ar** ausgewählt ist aus:
3'-Benzoylphenyl, 3'-(4-Chlor-benzoyl)-phenyl, 3'-(4-Methyl-benzoyl)-phenyl, 3'-Acetyl-phenyl, 3'-Propionyl-phenyl, 3'-Isobutanoyl-phenyl, 4'-Isobutyl-phenyl, 4'-Trifluormethansulfonyloxy-phenyl, 4'-Benzolsulfonyloxy-phenyl, 4'-Trifluormethansulfonylamino-phenyl, 4'-Benzolsulfonylamino-phenyl, 4'-Benzolsulfonylmethyl-phenyl, 4'-Acetoxyphenyl, 4'-Propionyloxy-phenyl, 4'-Benzoyloxy-phenyl, 4'-Acetylamino-phenyl, 4'-Propionylamino-phenyl, 4'-Benzoylamino-phenyl, 3'-(Furan-2-carbonyl)-phenyl, 3'-(Benzofuran-2-carbonyl)-phenyl, 3'-(Thiophen-2-carbonyl)-phenyl, 3'-(Pyridin-2-carbonyl)-phenyl, 3'-(Thiazol-2-carbonyl)-phenyl, 3'-(Oxazol-2-carbonyl)-phenyl, 3'-(2-Furyl)-phenyl, 3'-(2-Oxazolyl)-phenyl, 3'-(3-Isoxazolyl)-phenyl, 3'-(2-Benzoxazolyl)-phenyl, 3'-(3-Benzoisoxazolyl)-phenyl, 3'-(2-Thiazolyl)-phenyl, 3'-(2-Pyridyl)-phenyl, 3'-(2-Thiophenyl)-phenyl;
oder Ar ist ein Heteroarylring, ausgewählt aus Pyridin, Pyrrol, Thiophen, Furan oder Indol.

12. C5aR1-Inhibitor zur Verwendung nach den Ansprüchen 1 bis 8 und 11, wobei die Verbindung ausgewählt ist aus:
- (2R)-2-[3-(Furan-2-carbonyl)phenyl]-N-[4-(trifluormethyl)-1,3-thiazol-2-yl]propenamid (DF2427);
- 4-{(1R)-1-[(Phenylsulfonyl)amino]ethyl} phenyltrifluormethansulfonat;
- N-[(1R)-1-(3-Benzoylphenyl)ethyl]benzolsulfonamid;
- 4-{(1R)-1-[(Pyridin-3-ylsulfonyl)amino]ethyl}phenyltrifluormethansulfonat;
- N-[(1R)-1-(3-Benzoylphenyl)ethyl]methansulfonamid;
- N-{(1R)-1-[3-(2-Furoyl)phenyl]ethyl}thiophen-2-sulfonamid;
- N-{(1R)-1-[3-(2-Furoyl)phenyl]ethyl}methansulfonamid;
- 4-{(1R)-1-[(Thien-2-ylsulfonyl)amino]ethyl} phenyltrifluormethansulfonat;
- N-[(1R)-1-(3-Benzoylphenyl)ethyl]thiophen-2-sulfonamid;
- N-[(1R)-1-(3-Benzoylphenyl)ethyl]-3-pyrrolidin-1-ylpropan-1-sulfonamid;
- Methyl-5-({[(1R)-1-(3-benzoylphenyl)ethyl]amino}sulfonyl)-2-furoat;
- 5-({[(1R)-1-(3-Benzoylphenyl)ethyl]amino}sulfonyl)-2-fursäure;
- 4-{(1R)-2-Methyl-1-[(methylsulfonyl)amino]propyl}phenyltrifluormethansulfonat;
- N-((1R)-1-{4-[1-Methyl-1-(phenylsulfonyl)ethyl]phenyl}ethyl)methansulfonamid;
- 4-[(1R)-1-(Isobutyrylamino)ethyl]phenyltrifluormethansulfonat;
- 4-{[(1R)-1-(Pyridin-3-ylcarbonyl)amino]ethyl]}phenyltrifluormethansulfonat;
- N-[(1R)-1-(3-Benzoylphenyl)ethyl]benzamid;
- N-[(1R)-1-(3-Benzoylphenyl)ethyl]-2-furamid;
- N-[(1R)-1-(3-Benzoylphenyl)ethyl]cyclobutancarboxamid;
- N-[(1R)-1-(4-Trifluormethansulfonyloxy)phenylethyl]-4-piperidin-1-ylbutanamid (DF2593Y);
- 4-{(1R)-1-[(4-Pyrrolidin-1-ylbutanoyl)amino]ethyl]}phenyltrifluormethansulfonat;
- 3-{(1R)-1-[4-(4-Trifluormethyl-1,3-thiazol-2-yl)amino]ethyl}phenyl) (phenyl)methanon;
- R(-)-2-[(4'-Trifluormethansulfonyloxy)phenyl]-N-[3-(N'-pirrolidinyl)propyl] propionamid (DF2297X) oder dessen Chloridsalz (DF2297A); und
- 5-[(1R)-1-(4-{[4-(Trifluormethyl)-1,3-thiazol-2-yl]amino}phenyl)ethyl] tetrazol-2-id (DF3966Y) oder dessen Natriumsalz (DF3966A).

13. Pharmazeutische Zusammensetzung, umfassend einen C5aR1-Inhibitor wie in den Ansprüchen 1 bis 12 beschrieben und mindestens einen inerten pharmazeutisch verträglichen Hilfsstoff zur Verwendung bei der Prävention oder Behandlung einer Überempfindlichkeitsreaktion (HSR) gegen ein Taxan bei einem Individuum.

## Revendications

1. Inhibiteur de C5aR1 destiné à être utilisé pour la prévention ou le traitement d'une réaction d'hypersensibilité (HSR) à un taxane chez un individu.

2. Inhibiteur de C5aR1 destiné à être utilisé selon la revendication 1, dans lequel ledit taxane est choisi parmi le paclitaxel, le Nab^{™}-paclitaxel, le docétaxel et le cabazitaxel.

3. Inhibiteur de C5aR1 destiné à être utilisé selon la revendication 1 ou 2, dans lequel ledit inhibiteur de C5aR1 est destiné à être utilisé pour la prévention d'une réaction d'hypersensibilité à un taxane chez ledit individu et l'inhibiteur est administré une ou plusieurs fois avant chaque administration dudit taxane.

4. Inhibiteur de C5aR1 destiné à être utilisé selon la revendication 3, dans lequel ledit inhibiteur de C5aR1 est administré une ou plusieurs fois entre 30 minutes et 24 heures avant l'administration du taxane à l'individu.

5. Inhibiteur de C5aR1 destiné à être utilisé selon la revendication 3 ou 4, dans lequel ledit individu est un patient cancéreux qui a déjà eu un épisode d'HSR à un taxane, qui a eu un résultat positif à un test cutané de sensibilité audit taxane et/ou qui a des antécédents d'atopie.

6. Inhibiteur de C5aR1 destiné à être utilisé selon les revendications 3 à 5, dans lequel il est administré en association avec un ou plusieurs corticostéroïdes et/ou antihistaminiques.

7. Inhibiteur de C5aR1 destiné à être utilisé selon la revendication 1 ou 2, dans lequel ledit inhibiteur de C5aR1 est destiné à être utilisé dans le traitement aigu d'une réaction d'hypersensibilité à un taxane chez ledit individu et l'inhibiteur est administré dès l'apparition des symptômes de la réaction d'hypersensibilité.

8. Inhibiteur de C5aR1 destiné à être utilisé selon les revendications 1 à 7, dans lequel ledit inhibiteur de C5aR1 est choisi parmi les antagonistes compétitifs de C5aR1, les anticorps anti-C5aR1 peuvent bloquer les sites de liaison de C5a sur le récepteur et les inhibiteurs allostériques non compétitifs de C5aR1.

9. Inhibiteur de C5aR1 destiné à être utilisé selon les revendications 1 à 8, qui est un antagoniste de C5aR1 choisi dans le groupe constitué par :
- (2R,3S)-2-[4-(Cyclopentylamino)phényl]-1-(2-fluoro-6-méthylbenzoyl)-N-[4-méthyl-3-(trifluorométhyl)phényl]pipéridine-3-carboxamide (Avacopan, Vynpenta^{®}) ;
- N-acétyl-L-phénylalanyl-L-ornithyl-L-prolyl-3-cyclohexyl-D-alanyl-L-tryptophyl-L-arginine-N-5.2-C-1.6-lactame (PMX-53) ;
- Phénylalanine acétylée-[ornithyl-proline-(D)cyclohexylalanine-tryptophyl-arginine] ;
- L-Alanyl-L-séryl-glycyl-L-alanyl-L-prolyl-L-alanyl-L-prolyl-glycyl-L-prolyl-L-alanyl-glycyl-L-prolyl-L-leucyl-L-arginyl-L-prolyl-L-méthionyl-L-phénylalanine ;
- N,N-Bis(1,3-benzodioxol-5-ylméthyl)-N-(1-butyl-2,4-diphényl-1H-imidazol-5-ylméthyl)amine ;
- N-[2-(4-Chlorophényl)éthyl]-N-(1,4-dioxaspiro[4.5]dec-8-yl)-2-isobutylbenzamide ;
- N-[2-(4-Chlorophényl)éthyl]-N-(4-hydroxycyclohexyl)-1-benzothiophène-3-carboxamide ;
- N-[2-(4-Chlorophényl)éthyl]-N-(4-hydroxycyclohexyl)naphtalène-1-carboxamide ;
- 2-(2-Éthyl-6-méthylphényl)-4-méthoxy-N-(5-méthoxy-2-méthylphényl)-5,6,7,8-tétrahydroquinoline-5-amine ;
- 2-(2,6-Diéthylphényl)-N-éthyl-4-méthoxy-N-(1-naphthyl)-5,6,7,8-tétrahydroquinoline-5-amine ;
- N-[2,6-Dioxohexahydropyrimidine-4(S)-ylcarbonyl]-L-phénylalanyl-L-ornithyl-L-prolyl-5-méthyl-L-norleucyl-4-fluoro-L-phénylalanyl-L-phénylalaninamide (JPE-1375 ; JSM-1375) ;
- N-(3-Phénylpropionyl)-L-ornithyl-L-prolyl-3-cyclohexyl-D-alanyl-L-tryptophyl-L-arginine N-5.1-C-1.5-lactame (PMX-205) ;
- N,N'-Bis(4-amino-2-méthylquinoline-6-yl)urée (NSC12155) ;
- Chlorhydrate de N-[4-(Diméthylamino)benzyl]-N-(4-isopropylphényl)-7-méthoxy-1,2,3,4-tétrahydronaphtalène-1-carboxamide (W54011) ;
- Peptide L-phénylalanyl-L-ornithyl-L-prolyl-3-cyclohexyl-D-alanyl-L-tryptophyl-L-arginine N-5.2-C-1 .6-cyclique ;
- (4aR,16aS)-6,18-Dihydroxy-23(S)-[2(S)-hydroxy-2-[2(R)-hydroxy-6(R)-méthyl-5(R)-[2(S)-méthylbutyl]tétrahydro-2H-pyran-2-yl]propionamido]-22(S)-isopropyl-7(S),19(R)-diméthyldocosahydro-13H,22H-dipyridazino[6,1-f :6',1'-o][1,4,7,10,13,16]oxapentaazacyclononadécine-5,7,11,17,20,24-hexanone (L-156602).

10. Inhibiteur de C5aR1 destiné à être utilisé selon les revendications 1 à 8, qui est un anticorps anti-C5aR1 choisi dans le groupe constitué par :
- Avdoralimab (IPH-5401)
- MOR-044254 ;
- NOX-020 ;
- Anti-C5aR1 ab-C5-SiRNA ;
- m20/70 mlgG2a.1 ;
- 3C5 ;
- 7F3.

11. Inhibiteur de C5aR1 destiné à être utilisé selon les revendications 1 à 8, qui est un inhibiteur allostérique non compétitif choisi parmi
- (2*R*)-2-[3-(furan-2-carbonyl)phényl]-*N*-[4-(trifluorométhyl)-1,3-thiazol-2-yl]propénamide (DF2427) ; et
- des composés répondant à la formule générale (I) 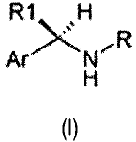
ou un sel pharmaceutiquement acceptable de celui-ci, dans lequel
i) **R** est choisi parmi :
- 2-thiazolyl ou 2-oxazolyl, non substitué ou substitué par un groupe choisi parmi les groupes méthyle, tert-butyle ou trifluorométhyle ;
- C(Ra)=N-W dans lequel W est un alkyle linéaire ou ramifié en C₁-C₄,
- CORa, SORa, SO₂Ra, PORa, PO₂Ra,
dans lequel
Ra est choisi parmi
- l'alkyle en C₁-C₅, le cycloalkyle en C₃-C₆, l'alcényle en C₂-C₅, le phényle non substitué ou substitué par un groupe choisi parmi les halogènes, l'alkyle en C₁-C₄, l'alkoxy en C₁-C₄, l'halo-alkoxy en C₁-C₄, l'hydroxy, l'acyloxy en C₁-C₄, le phénoxy, le cyano, le nitro, l'amino ;
- un groupe hétéroaryle choisi parmi la pyridine, la pyrimidine, le pyrrole, le thiophène, le furane, l'indole, le thiazole, l'oxazole, cet hétéroaryle étant non substitué ou substitué par un groupe choisi parmi l'halogène, l'alkyl en C₁-C₄, l'alkoxy en C₁-C₄, l'halo-alkoxy en C₁-C₄, l'hydroxy, l'acyloxy en C₁-C₄, le phénoxy, le cyano, le nitro, l'amino ;
- un résidu α ou β carboxyalkyle constitué d'un alkyl linéraire ou ramifié en C₁-C₆, d'un cycloalkyl en C₃-C₆, d'un alcényle en C₂-C₆, d'un phénylalkyle en C₁-C₆, éventuellement substitué par un autre groupe carboxy (COOH) ;
- un groupe ω-aminoalkylamino de formule (II) : 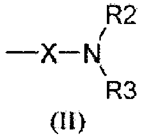 dans lequel, dans ladite formule (II)
X est :
- un alkylène linéaire ou ramifié en C₁-C₆, un alcénylène en C₄-C₆, un alcynylène en C₄-C₆, éventuellement substitué par : a) un groupe CO₂R4, dans lequel R4 représente l'hydrogène ou un groupe alkyle linéaire ou ramifié en C₁-C₆ ou un groupe alcényle linéaire ou ramifié en C₂-C₆, ou b) par un groupe CONHR5 dans lequel R5 représente l'hydrogène, un groupe alkyle linéaire ou ramifié en C₂-C₆ ou un groupe OR4, R4 étant défini comme ci-dessus ;
- un groupe (CH₂)ₘ-B-(CH₂)ₙ, éventuellement substitué par un groupe CO₂R4 ou CONHR5, tel que défini ci-dessus, dans lequel a) B est un atome d'oxygène, de soufre ou d'azote éventuellement substitué par un groupe alkyle en C₁-C₄, m est égal à zéro ou à un nombre entier de 2 à 3 et n est un nombre entier de 2 à 3, ou b) B est un groupe CO, SO ou CONH, m est un nombre entier de 1 à 3 et n est un nombre entier de 2 à 3 ;
R2 et R3 sont indépendamment un hydrogène, un alkyle linéaire ou ramifié en C₁-C₆, éventuellement interrompu par un atome d'oxygène ou de soufre, un cycloalkyle en C₃-C₇, un alcényle en C₃-C₅, un alcynyle en C₃-C₅, un groupe aryle-alkyle en C₁-C₃, un groupe hydroxy-alkyle en C₂-C₃ ; ou
R2 et R3 forment, avec l'atome N auquel ils sont liés, un cycle hétérocyclique azoté de 3 à 7 membres de formule (III) 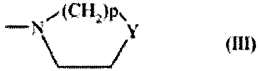 dans lequel
Y représente :
- une liaison simple,CH₂, O, S, ou un groupe N-R6, où R6 représente l'hydrogène, un alkyle en C₁-C₄, un acyle en C₁-C₄, un phényle non substitué ou substitué par un groupe choisi parmi l'halogène, l'alkyle en C₁-C₄, l'alcoxy en C₁-C₄, l'hydroxy, l'acyloxy en C₁-C₄, le phénoxy, le cyano, le nitro, l'amino,
et p représente un nombre entier de 0 à 3 ;
- un résidu de formule SO₂R7 dans laquelle R7 est un alkyle en C₁-C₆, un cycloalkyle en C₃-C₆, un alcényle en C₂-C₆, un aryle ou un hétéroaryle ;
ou
dans ladite formule (II)
X forme, avec l'atome d'azote auquel il est lié et avec le groupe R2, un cycle hétérocyclique, monocyclique ou polycyclique de 3 à 7 membres contenant de l'azote, et **R3** représente l'hydrogène, l'alkyle en C₁-C₄, l'acyle en C₁-C₄, le phényle non substitué ou substitué par un groupe choisi parmi l'halogène, l'alkyle en C₁-C₄, l'alcoxy en C₁-C₄, l'hydroxy, l'acyloxy en C₁-C₄, le phénoxy, le cyano, le nitro, l'amino ;
ii) **R1** est un alkyle linéaire ou ramifié en C₁-C₅, un cycloalkyle en C₃-C₅;
iii) **Ar est**
- un groupe phényle non substitué ou substitué par un ou plusieurs groupes choisis indépendamment parmi l'halogène, l'alkyle en C₁-C₄, l'alcoxy en C₁-C₄, l'hydroxy, l'acyloxy en C₁-C₄, le phénoxy, le cyano, le nitro, l'amino, l'acylamino en C₁-C₄, l'halo-alkyle en C₁-C₃, l'halo-alkoxy en C₁-C₃, le benzoyle, le carbonyle hétéroaryle, l'hétéroaryle, l'alcanesulfonate linéaire ou ramifié en C₁-C₈, les alcanesulfonamides linéaires ou ramifiés en C₁-C₈, le sulfonylméthyle linéaire ou ramifié en C₁-C₈ ; ou
- un cycle hétéroaryle choisi parmi la pyridine, le pyrrole, le thiophène, le furane et l'indole.
Parmi les composés ci-dessus, les composés de la formule (I) ou leurs sels pharmaceutiquement acceptables sont particulièrement préférés, dans lesquels :
R est choisi parmi :
- 2-thiazolyl ou 2-oxazolyl, non substitué ou substitué par un groupe choisi parmi les groupes méthyle, tert-butyle ou trifluorométhyle ;
- C(Ra)=N-W dans lequel W est un alkyle linéaire ou ramifié en C₁-C₄,
- CORa, SORa ou SO₂Ra,
dans lequel Ra est tel que défini ci-dessus ; et
**Ar** est choisi parmi :
3'-benzoylphényl, 3'-(4-chloro-benzoyl)-phényl, 3'-(4-méthyl-benzoyl)-phényl, 3'-acétyle-phényl, 3'-propionyl-phényl, 3'-isobutanoyl-phényl, 4'-isobutyl-phényl, 4'-trifluorométhanesulfonyloxy-phényl, 4'-benzènesulfonyloxy-phényl, 4'-trifluorométhanesulfonylamino-phényl, 4'-benzènesulfonylamino-phényl, 4'-benzènesulfonylméthyl-phényl, 4'-acétoxyphényl, 4'-propionyloxy-phényl, 4'-benzoyloxy-phényl, 4'-acétylamino-phényl, 4'propionylamino-phényl, 4'-benzoylamino-phényl, 3'-(furan-2-carbonyl)-phényl, 3'-(benzofuran-2-carbonyl)-phényl, 3'-(thiophène-2-carbonyl)-phényl, 3'-(pyridine-2-carbonyl)-phényl, 3'-(thiazole-2-carbonyl)-phényl, 3'-(oxazole-2-carbonyl)-phényl, 3'-(2-furyl)-phényl, 3'-(2-oxazolyl)-phényl, 3'-(3-isoxazolyl)-phényl, 3'-(2-benzoxazolyl)-phényl, 3'-(3-benzoisoxazolyl)-phényl, 3'-(2-thiazolyl)-phényl, 3'-(2-pyridyl)-phényl, 3'-(2-thiophényl)-phényl ;
ou Ar est un cycle hétéroaryle choisi parmi la pyridine, le pyrrole, le thiophène, le furane ou l'indole.

12. Inhibiteur de C5aR1 destiné à être utilisé selon les revendications 1 à 8 et 11, dans lequel ledit composé est choisi parmi :
- (2R)-2-[3-(furan-2-carbonyl)phényl]-N-[4-(trifluorométhyl)-1,3-thiazol-2-yl]propénamide (DF2427) ;
- 4-{(1R)-1-[(phénylsulfonyl)amino]éthyl}phényl trifluorométhanesulfonate ;
- N-[(1R)-1-(3-benzoylphényl)éthyl]benzènesulfonamide ;
- 4-{(1R)-1-[(pyridine-3-ylsulfonyl)amino]éthyl}phényltrifluorométhanesulfonate ;
- N-[(1R)-1-(3-benzoylphényl)éthyl]méthanesulfonamide ;
- N-{(1R)-1-[3-(2-furoyl)phényl]éthyl}thiophène-2-sulfonamide ;
- N-{(1R)-1-[3-(2-furoyl)phényl]éthyl}méthanesulfonamide ;
- 4-{(1R)-1-[(thien-2-ylsulfonyl)amino]éthyl}phényl trifluorométhanesulfonate ;
- N-[(1R)-1-(3-benzoylphényl)éthyl]thiophène-2-sulfonamide ;
- N-[(1R)-1-(3-benzoylphényl)éthyl]-3-pyrrolidine-1-ylpropane-1-sulfonamide ;
- méthyl 5-({[(1R)-1-(3-benzoylphényl)éthyl]amino}sulfonyl)-2-furoate ;
- Acide 5-({[(1R)-1-(3-benzoylphényl)éthyl]amino}sulfonyl)-2-furoïque ;
- 4-{(1R)-2-méthyl-1-[(méthylsulfonyl)amino]propyl}phényltrifluorométhanesulfonate ;
- N-((1R)-1-{4-[1-méthyl-1-(phénylsulfonyl)éthyl]phényl}éthyl)méthanesulfonamide ;
- 4-[(1R)-1-(isobutyrylamino)éthyl]phényltrifluorométhanesulfonate ;
- 4-{[(1R)-1-(pyridine-3-ylcarbonyl)amino]éthyl]}phényltrifluorométhanesulfonate ;
- N-[(1R)-1-(3-benzoylphényl)éthyl]benzamide ;
- N-[(1R)-1-(3-benzoylphényl)éthyl]-2-furamide ;
- N-[(1R)-1-(3-benzoylphényl)éthyl]cyclobutanecarboxamide ;
- N-[(1R)-1-(4-trifluorométhanesulfonyloxy)phényléthyl]-4-pipéridine-1-yl butanamide (DF2593Y) ;
- 4-{(1R)-1-[(4-pyrrolidine-1-ylbutanoyl)amino]éthyl]}phényl trifluorométhanesulfonate ;
- 3-{(1R)-1-[4-(4-trifluorométhyl-1,3-thiazol-2-yl)amino]éthyl}phényl) (phényl)méthanone ;
- R(-)-2-[(4'-trifluorométhanesulfonyloxy)phényl]-N-[3-(N'-pirrolidinyle)propyl] propionamide (DF2297X) ou son sel de chlorure (DF2297A) ; et
- 5-[(1R)-1-(4-{[4-(trifluorométhyl)-1,3-thiazol-2-yl]amino}phényl)éthyl]tétrazol-2-ide (DF3966Y) ou son sel de sodium (DF3966A).

13. Composition pharmaceutique comprenant un inhibiteur de C5aR1 tel que décrit dans les revendications 1 à 12 et au moins un excipient inerte pharmaceutiquement acceptable, destiné à être utilisé pour la prévention ou le traitement d'une réaction d'hypersensibilité (HSR) à un taxane chez un individu.
